# EUROPEAN PATENT APPLICATION

(11) **EP 4 582 547 A1**
(43) Date of publication of application: **09.07.2025**
(21) Application number: 23860423.5
(22) Date of filing: 30.08.2023
(51) Int. Cl.: C12P 1/04, C12P 13/14, C12N 1/00, C12N 1/20, C12N 1/21, C12N 15/52, C12N 15/54

(54) **METHOD FOR FERMENTATIVELY PRODUCING L-GLUTAMIC ACID DERIVED FROM NITROGEN MOLECULE**

(30) Priority: 30.08.2022 JP 2022137333; 03.03.2023 JP 2023033168
(71) Applicant: Kikkoman Corporation, Noda-shi, Chiba 278-8601 (JP); The University of Tokyo, Bunkyo-ku, Tokyo 113-8654 (JP)
(72) Inventor: YOSHIDOME, Daisuke, Tokyo 113-8654 (JP); ITO,Yusuke, Noda-shi, Chiba 278-8601 (JP); YAMASHITA, Kona, Noda-shi, Chiba 278-8601 (JP); HIDAKA, Makoto, Tokyo 113-8654 (JP); MATSUDA KOSONO, Saori, Tokyo 113-8654 (JP); NISHIYAMA, Makoto, Tokyo 113-8654 (JP)
(74) Representative: Gill Jennings & Every LLP
(86) International application number: PCT/JP2023/031570
(87) International publication number: WO 2024/048661

(57) **Abstract**

The purpose of the present invention is to develop a method for fermentatively producing L-glutamic acid based on a nitrogen molecule in air using nitrogen-fixing bacteria. A culture method, by which a state in which the nitrogen-fixing bacteria have enhanced nitrogen fixation ability is maintained, has been developed. The culture method is characterized by containing an organic acid such as citric acid in a nitrogen-limited medium put in an non-hermetic vessel. A method for fermentatively producing L-glutamic acid dependent on nitrogen fixation could be developed by culturing genetically modified strains, which highly express a citric acid synthase gene and a citric acid transporter gene, by means of the culture method. A method for fermentatively producing L-glutamic acid dependent on nitrogen fixation could be developed by using modified strains, which highly express a citric acid synthase gene or a 2-methylcitric acid synthase gene, in the process to reach the aforementioned step. The present invention pertains to a production method and a culture method for such microorganisms.

## Description

### FIELD

The present invention relates to a culture medium that allows the nitrogen fixing ability of nitrogen-fixing bacteria to be maximally exhibited, and to a culturing method using the above culture medium, as well as to fermentative production of L-glutamic acid using nitrogen molecules in the atmosphere which implements the above culture medium and culturing method, and to a microorganism capable of fermentation of L-glutamic acid using nitrogen molecules in the atmosphere.

### BACKGROUND

Certain microorganisms have the ability to convert nitrogen molecules in the atmosphere to ammonia. Such ability is referred to as nitrogen fixing ability. The ongoing search for microorganisms with nitrogen fixing ability has led to the discovery of several hundred microorganic species, and since all of them are microorganisms belonging to the Bacteria domain (Eubacteria) or the Archaea domain (Archaebacteria), they are collectively referred to as nitrogen-fixing bacteria. Nitrogen-fixing bacteria are able to grow with nitrogen molecules in the air as their sole nitrogen source.

The enzymes involved in nitrogen fixiation are nitrogenases, which catalyze the following reaction under ideal reacting conditions *in vitro.*

N₂ + 8H⁺ + 8e⁻ + 16ATP → 2NH₃ + H₂ + 16ADP + 16Pi

Nitrogenases are complex enzymes comprising dinitrogenase, which reduces nitrogen molecules, and dinitrogenase reductase which transfers electrons to dinitrogenase. Dinitrogenase has three subtypes, the Mo-type, V-type and Fe-type, and all nitrogen-fixing bacteria possess the Mo-type dinitrogenase, which has an iron-molybdenum cofactor at the catalytic center. Nitrogen-fixing bacteria therefore cannot exhibit nitrogen fixing ability without a sufficient amount of molybdenum and iron present in the environment. Furthermore, if nitrogen sources (ammonium salts, nitrates and protein degradation products such as peptones) are present in the environment, nitrogen-fixing bacteria do not exhibit nitrogen fixing ability but rather assimilating those nitrogen sources for their growth. In other words, nitrogen-fixing bacteria conduct nitrogen fixation only when nitrogen sources have been depleted from the environment.

Among nitrogen-fixing bacteria, cyanobacteria produce the electrons and ATP used by dinitrogenase reductase via photosynthesis and are therefore able to fix nitrogen autotrophically. In contrast, heterotrophic nitrogen-fixing bacteria produce the electrons and ATP used by dinitrogenase reductase via the metabolism of carbon sources supplied by the plant in the natural world. Nitrogen-fixing bacteria can be categorized, based on their association with plants, into (root-stem) nodule-forming types, rhizospheric types and endophytic types. Some nodule-forming nitrogen-fixing bacteria are only able to exhibit nitrogen fixing ability in nodules, but other nitrogen-fixing bacteria can be cultured in test tubes while exhibiting nitrogen fixing ability using nitrogen-free medium (synthetic minimal medium containing no nitrogen sources). However, the sugars or organic acids preferred as carbon sources differ for each type of nitrogen-fixing bacteria. Whether nitrogen fixation is conducted under anaerobic conditions, microaerobic conditions or aerobic conditions reflects the pathway through which electrons are transferred to dinitrogenase reductase, which also differs in different nitrogen-fixing bacteria. These factors work in combination, so that a wide variety of methods exist for culturing nitrogen-fixing bacteria exhibiting nitrogen fixing ability. For example, in most cases nitrogen fixing ability is exhibited by *Klebsiella oxytoca* with sugar-supplied anaerobic culturing, by *Azospirillum lipoferum* with organic acid-supplied microaerobic culture, and by *Azotobacter vinelandii* with sugar-supplied aerobic culturing.

When culturing is initiated by inoculating nitrogen-fixing bacteria into liquid nitrogen-free medium (at about 10⁶ cells/mL, for example), the cell growth accompanying nitrogen fixing ability is exhibited after a long lag time, and a culturing period of several weeks is therefore required to observe phenomena that are related to nitrogen fixing ability. When nitrogen-fixing bacteria are spread onto nitrogen limited agar comprising nitrogen-free medium with addition of 100 mg/L yeast extract, they rapidly formed colony dependent on nitrogen fixing ability. Colonies exhibiting nitrogen fixing ability may sometimes be obtained even by culturing on solid medium in air. One group of the nitrogen-fixing bacteria is belonging to *Klebsiella* spp. (NPL 1: Rennie, Can. J. Microbiol. 27 8-14 1981). It was therefore considered that it might be possible to exhibit stable nitrogen fixing ability even with static culture of *Klebsiella oxytoca* with an nitrogen-limited liquid medium containing 100 mg/L yeast extract in non-hermetic vessel with air, and such a culturing method was established. In this culturing method it was found that nitrogen-fixing bacteria begin to grow with virtually no lag time, exhibiting nitrogen fixing ability in 24 hours after the start of culturing, and completing nitrogen fixing ability-related phenomena within a one-week culturing period.

The acetylene reduction method is a convenient method for measuring the nitrogen fixing ability. Because nitrogenases have low substrate specificity, they also reduce acetylene to ethylene (acetylene reducing activity). When nitrogen-fixing bacteria are cultured with a gaseous mixture of nitrogen molecules and acetylene, the nitrogen-fixing bacteria reduce both, simultaneously producing ammonia and ethylene. Ammonia is consumed by nitrogen assimilation, making it impossible to measure nitrogen fixing activity in real time. Ethylene, on the other hand, is released into the air layer without being further metabolized. The acetylene reducing activity of nitrogenases is therefore determined by measuring the amount of ethylene accumulated in the air layer. The nitrogen fixing ability of nitrogen-fixing bacteria is represented as the value of the acetylene reducing activity.

L-glutamic acid is an important amino acid used in foods and pharmaceuticals. Microorganisms have the ability to produce L-glutamic acid from ammonium and 2-oxoglutaric acid, either by L-glutamate dehydrogenase or the glutamine synthetase-glutamate synthase complex system. This metabolic process is referred to as "nitrogen assimilation". Methods have been developed to date for fermentatively producing L-glutamic acid using a variety of genetically modified microorganisms. Such genetic modification is fundamentally modification that increases the carbon flux to2-oxoglutaric acid which is the recipient of ammonium ions in nitrogen assimilation. In PTL 1: Japanese Patent Publication No. 4144131, for example, *Klebsiella* bacteria are modified to induce high expression of citrate synthase (CS) derived from *Corynebacterium* bacteria, and fermentative production of L-glutamic acid is carried out by aerobic culturing of the modified *Klebsiella* bacteria in culture medium containing a large amount of ammonium ion (20 g/L ammonium sulfate). PTL 2: Japanese Unexamined Patent Publication No. 2009-254323 reports a method for fermentatively producing L-glutamic acid wherein *Klebsiella* bacteria are modified to induce high expression of methylcitrate synthase (MCS) derived from *E. coli* or *Corynebacterium* bacteria, and anaerobic culturing of the modified *Klebsiella* bacteria is carried out in culture medium containing a large amount of ammonium ion (10 g/L ammonium sulfate). In these examples, a large amount of ammonium ion is introduced into the culture medium for nitrogen assimilation. The commonly used method for producing ammonia is currently the Haber-Bosch process. In the process, hydrogen gas produced by steam reforming of fossil fuels (coal, petroleum and methane gas) are reacted with nitrogen molecules in the atmosphere to produce ammonia, and it requires considerable cost for operation.

### [CITATION LIST]

### [PATENT LITERATURE]

[PTL 1] Japanese Patent Publication No. 4144131
[PTL 2] Japanese Unexamined Patent Publication No. 2009-254323

### [NON PATENT LITERATURE]

[NPL 1] Rennie, Can. J. Microbiol. 27 8-14 1981
[NPL 2] Bott, Mol. Microbiol. 18 533-546 1995
[NPL 3] Weitzman, Adv. Microb. Physiol. 22 185-244 1981
[NPL 4] Saier, Microbiol. Mol. Biol. Rev. 64 354-411 2000
[NPL 5] Soares-Silva et al., FEMS Microbiol. Letter. 367 1-15 2020
[NPL 6] Kaestner et al., Arch. Microbiol. 177 500-506 2002
[NPL 7] Son HF et al., PLoS ONE. 11(6): e0158402
[NPL 8] Choi, J. W. et al., Microb Cell Fact 14, 21 2015

### SUMMARY

### [TECHNICAL PROBLEM]

In consideration of the above, and after searching for culturing conditions that allow nitrogen-fixing bacteria to exhibit maximum nitrogen fixing ability, a novel fermentative production method was devised using nitrogen-fixing bacteria cultured under such conditions, as modified strains having augmented production of 2-oxoglutaric acid as in PTL 1 and PTL 2 (Japanese Patent Publication No. 4144131 and Japanese Unexamined Patent Publication No. 2009-254323). The object is to provide a novel fermentative production method for L-glutamic acid wherein ammonia formed by maximum nitrogen fixing ability is efficiently converted to L-glutamic acid.

### [SOLUTION TO PROBLEM]

Since the culturing conditions for exhibiting maximum nitrogen fixing ability differ greatly depending on the type of nitrogen-fixing bacteria, as mentioned above, medium compositions and culturing methods for *Klebsiella* microorganisms were examined. It was found that the nitrogen fixing ability of *Klebsiella* microorganisms is enhanced in nitrogen limited medium containing organic acids such as citric acid. It was also found that by introducing the citrate synthase (CS) gene or 2-methylcitrate synthase (2-MCS) gene into *Klebsiella* microorganisms and culturing in nitrogen limited medium containing an organic acid such as citric acid, these are able to produce L-glutamic acid or its salt from nitrogen molecules in the atmosphere. Moreover, when a citrate transporter gene, which was found as a novel gene, was introduced into *Klebsiella* microorganisms and they were cultured in nitrogen limited medium containing an organic acid such as citric acid, they produce L-glutamic acid or its salt from nitrogen molecules in the atmosphere. Moreover, *Klebsiella* microorganisms introduced both the citrate synthase gene and citrate transporter gene produced larger amounts of L-glutamic acid than by introduction of either gene alone. A method for fermentation production L-glutamic acid was finally established based on the nitrogen fixing ability of nitrogen-fixing bacteria, using a modified strain different from that of PTLs 1 and 2 (Japanese Patent Publication No. 4144131 and Japanese Unexamined Patent Publication No. 2009-254323), and the invention was completed.

The present invention relates to the following:
[1] A method for fermentatively producing L-glutamic acid or its salt from nitrogen molecules in the atmosphere, comprising culturing nitrogen-fixing bacteria to which at least one gene selected from the group consisting of citrate transporter, citrate synthase and 2-methylcitrate synthase has been introduced, in nitrogen limited medium containing an organic acid or its salt which is part of the TCA cycle.
[2] The method according to [1] above, wherein the nitrogen limited medium contains a nitrogen source at 3 mg/L to 1500 mg/L of yeast extract equivalent and/or 0.02 mM to 15 mM of ammonium ion equivalent.
[3] The method according to [1] or [2] above, wherein the organic acid or its salt is at a concentration of 0.5 g/L to 100 g/L.
[4] The method according to any one of [1] to [3] above, wherein the organic acid is citric acid, succinic acid, malic acid, fumaric acid or 2-oxoglutaric acid.
[5] The method according to any one of [1] to [4] above, wherein the nitrogen-fixing bacteria are microorganisms of Enterobacteriaceae.
[6] The method according to [5] above, wherein the nitrogen-fixing bacteria are microorganisms selected from the group consisting of *Klebsiella, Rahnella, Raoultella* and *Kosakonia* bacteria.
[7] Nitrogen-fixing bacteria selected from the group consisting of *Klebsiella, Rahnella, Kosakonia* and *Raoultella, wherein* the citrate transporter gene have been introduced thereto.
[8] Nitrogen-fixing bacteria according to [7] above, wherein the nitrogen-fixing bacteria of *Klebsiella* are selected from the group consisting of *Klebsiella oxytoca, Klebsiella michiganensis, Klebsiella grimontii, Klebsiella pasteurii, Klebsiella pneumoniae, Klebsiella variicola and Klebsiella indica,* the nitrogen-fixing bacteria of *Rahnella* is *Rahnella aquatilis,* or the nitrogen-fixing bacteria of *Raoultella* are selected from the group consisting of *Raoultella terrigena, Raoultella ornithinolytica* and *Raoultella planticola.*
[9] Nitrogen-fixing bacteria according to [7] above, wherein the nitrogen-fixing bacteria are *Klebsiella oxytoca* NG13 (Accession No.: NITE BP-03721), *Klebsiella indica* (JCM33718), *Klebsiella variicola* (JCM12419), Klebsiella sp. (NBRC100048, NBRC100441, NBRC109911), *Raoultella terrigena* (JCM1687 = ATCC33257), *Rahnella aquatilis* (JCM1683 = ATCC33071) and *Klebsiella planticola* (JCM20069 = ATCC8329).
[10] Nitrogen-fixing bacteria according to any one of [7] to [9] above, wherein the nitrogen-fixing bacteria fermentatively produces L-glutamic acid or its salt from nitrogen molecules under nitrogen limitation in the atmosphere.

### [ADVANTAGEOUS EFFECTS OF INVENTION]

Microorganisms constructed by the method of the invention produce L-glutamic acid while fixing nitrogen in the atmosphere. Fermentation of L-glutamic acid is thus possible without depending on ammonia produced by the Haber-Bosch method.

### BRIEF DESCRIPTION OF DRAWINGS

Fig. 1 is a diagram showing the full metabolic pathway for production of L-glutamic acid from nitrogen molecules in genetically modified NG13, as according to the invention.
Fig. 2 is a pair of graphs showing the results of LC-MS analysis of molecular weight of L-glutamic acid accumulated in culture medium after culturing of NG13-pCCS in ¹⁵N₂ and O₂ mixed gas or air.

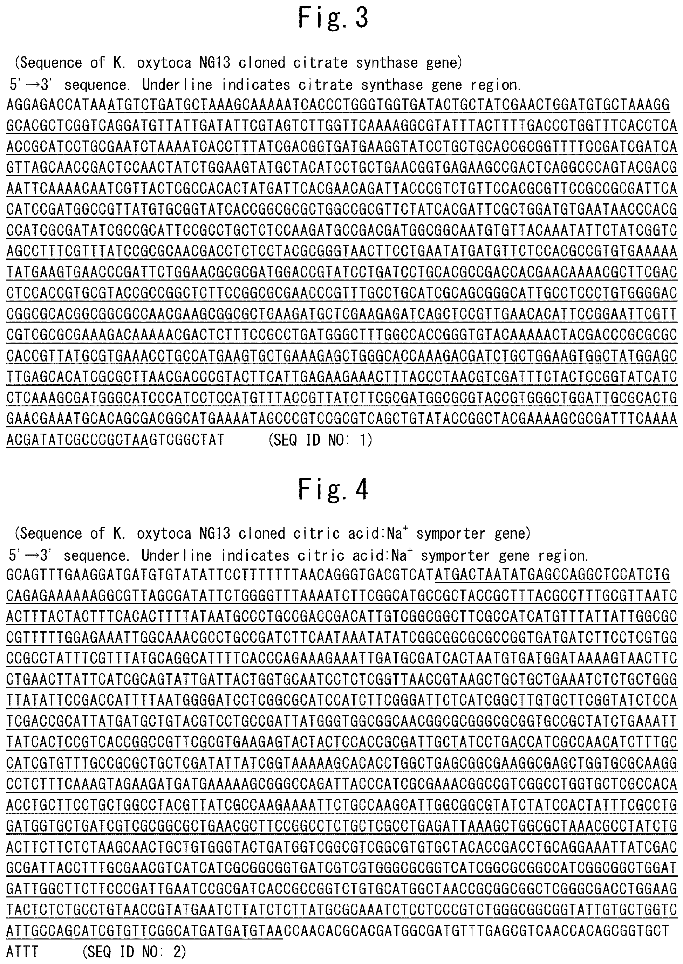

Fig. 3 shows the nucleotide sequence of the citrate synthase gene cloned in NG13.
Fig. 4 shows the nucleotide sequence of the citric acid:Na⁺ symporter gene cloned in NG13.

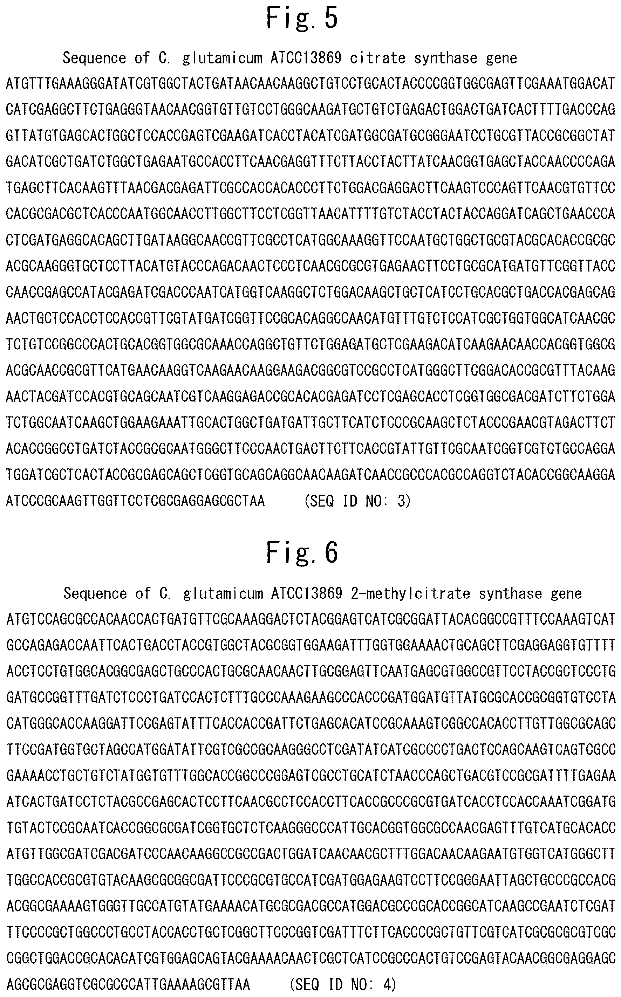

Fig. 5 shows the nucleotide sequence of the citrate synthase gene of *Corynebacterium glutamicum* ATCC13869.
Fig. 6 shows the nucleotide sequence of the 2-methylcitrate synthase gene of *Corynebacterium glutamicum* ATCC13869.

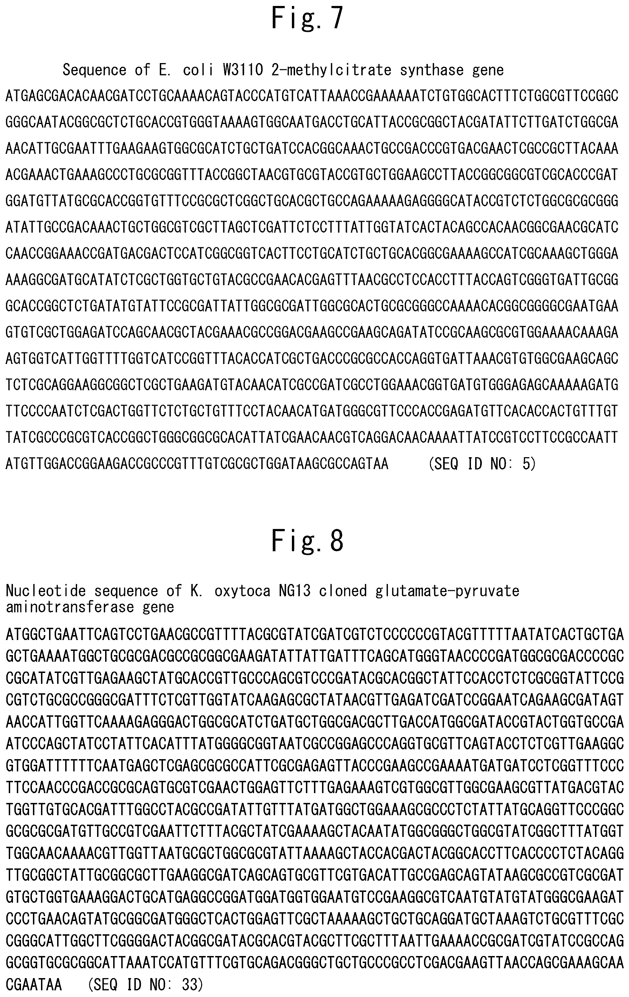

Fig. 7 shows the nucleotide sequence of the 2-methylcitrate synthase gene of *E. coli* W3110.
Fig. 8 shows the nucleotide sequence of the glutamate-pyruvate aminotransferase gene cloned in NG13.

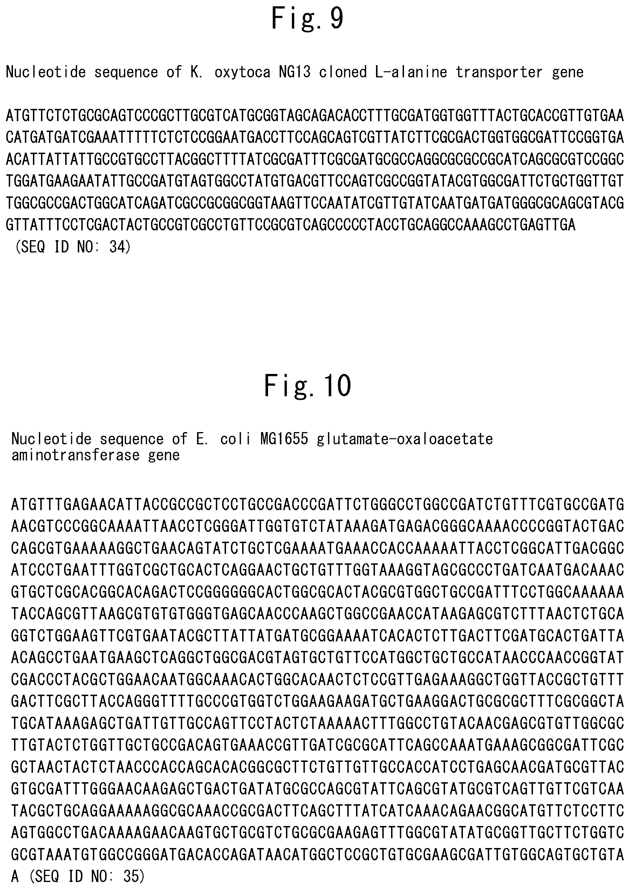

Fig. 9 shows the nucleotide sequence of the L-alanine transporter gene cloned in NG13.
Fig. 10 shows the nucleotide sequence of the glutamate-oxaloacetate aminotransferase gene of *E. coli* MG1655.

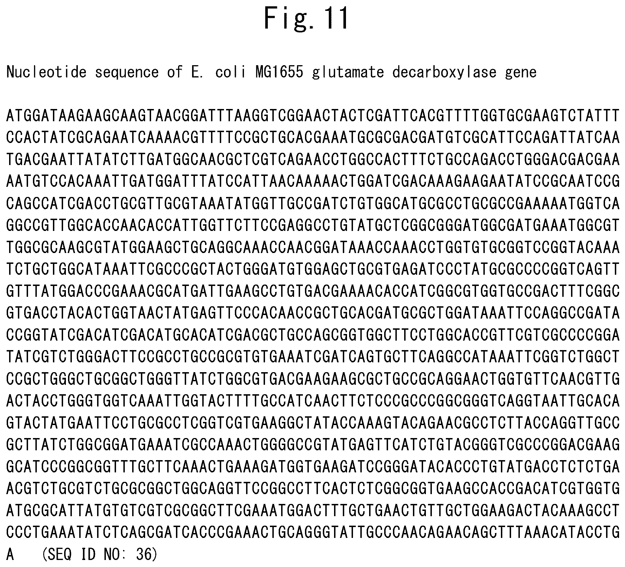

Fig. 11 shows the nucleotide sequence of the glutamate decarboxylase gene of *E. coli* MG1655.
Fig. 12 is a graph showing the results of analyzing the molecular weight of L-glutamic acid accumulated in culture medium after culturing *Rahnella* pCCS-pKCT in ¹⁵N₂ and O₂ mixed gas.

### DESCRIPTION OF EMBODIMENTS

One aspect of the invention relates to a method of culturing nitrogen-fixing bacteria while exhibiting nitrogen fixing ability in the atmosphere, and to culture medium in which the nitrogen-fixing bacteria maximally exhibits nitrogen fixing ability. Another aspect of the invention relates to nitrogen-fixing bacteria having a gene transferred so as to enhance production of L-glutamic acid, and to a method for fermentatively producing L-glutamic acid from nitrogen molecules in the atmosphere, the method comprising a step of culturing the nitrogen-fixing bacteria having a gene transferred so as to enhance production of L-glutamic acid, in nitrogen limited medium containing an organic acid such as citric acid.

### [Nitrogen limited medium]

For the purpose of the invention, "nitrogen limited medium" refers to culture medium with nitrogen content adjusted so as to cause nitrogen-fixing bacteria to exhibit nitrogen fixing ability. Nitrogen limited medium may also contain a nitrogen source at a concentration that enhances initial growth of nitrogen-fixing bacteria and does not inhibit nitrogen fixing activity. The nitrogen concentration of the nitrogen limited medium may vary depending on the type of nitrogen-fixing bacteria and the type of nitrogen source, and it may be represented in terms of yeast extract equivalent, for example. Since the amount of nitrogen required may vary depending on the type of nitrogen-fixing bacteria and the type of nitrogen source, as well as on the concentration of the carbon source, the amount of nitrogen that enhances initial growth of the nitrogen-fixing bacteria without inhibiting nitrogen fixing activity can be appropriately decided based on different culture conditions. As an example, the nitrogen limited medium of the invention may contain an organic acid such as citric acid or its salt, a nitrogen source at 50 mg/L to 300 mg/L and preferably 50 mg/L to 200 mg/L in terms of yeast extract equivalent, and also a carbon source separate from the organic acid such as citric acid or its salt. The nitrogen limited medium of the invention is suitable for culturing of nitrogen-fixing bacteria that fix nitrogen molecules in the atmosphere, and the nitrogen fixing ability can be further enhanced by citric acid or its salt.

The amount of nitrogen source in the nitrogen limited medium may vary depending on the nitrogen-fixing bacteria used. The nitrogen limited medium can be specified by the amount of nitrogen source in terms of yeast extract equivalent and/or in terms of ammonium ion equivalent. As an example, nitrogen limited medium can be prepared so as to contain a nitrogen source at 3 mg/L to 1500 mg/L in terms of yeast extract equivalent. As a more preferred nitrogen source amount, preparation may be so as to contain a nitrogen source at 25 mg/L to 500 mg/L in terms of yeast extract equivalent. As the most preferred nitrogen source amount, the nitrogen source may be adjusted to 50 mg/L to 300 mg/L in terms of yeast extract equivalent. When an ammonium salt is used as the nitrogen source in a nitrogen limited medium, the ammonium ion concentration may be adjusted to 0.02 mM to 15 mM. The concentration of ammonium ion as the nitrogen source in the nitrogen limited medium is preferably 0.2 mM to 5 mM and most preferably 0.35 mM to 3 mM. Nitrogen in the nitrogen limited medium is consumed by cells during initial growth of the nitrogen-fixing bacteria, and when the nitrogen concentration in the culture medium falls below a certain concentration, the nitrogen-fixing bacteria begin to fix nitrogen in the atmosphere, thereby producing L-glutamic acid or its salt from nitrogen molecules in the atmosphere. In other words, the nitrogen atoms forming L-glutamic acid that is fermentatively produced according to the invention include nitrogen atoms from nitrogen molecules in the atmosphere.

The nitrogen sources used may be extracts including yeast extract, amino acid mixtures including casamino acid and peptone, or ammonium salts such as ammonium chloride and ammonium sulfate. The concentration of the nitrogen source used in the nitrogen limited medium may be represented as the concentration in terms of yeast extract equivalent. Since yeast extract contains about 10% total Kjeldahl nitrogen, the concentration equivalent of yeast extract equivalent can be calculated based on the amount of total Kjeldahl nitrogen in the nitrogen source used. At the start of culturing, the nitrogen limited medium of the invention may usually contain the nitrogen source at 3 mg/L to 1500 mg/L in terms of yeast extract equivalent, or 0.3 mg/L to 150 mg/L as total Kjeldahl nitrogen. From the viewpoint of maximizing nitrogen fixing ability, the medium contains the nitrogen source at preferably 25 mg/L or greater in terms of yeast extract equivalent, or 2.5 mg/L or greater as total Kjeldahl nitrogen, more preferably 50 mg/L or greater in terms of yeast extract equivalent, or 5 mg/L or greater as total Kjeldahl nitrogen, and also preferably 500 mg/L or lower in terms of yeast extract equivalent, or 50 mg/L or lower as total Kjeldahl nitrogen, and more preferably 300 mg/L or lower in terms of yeast extract equivalent, or 30 mg/L or lower as total Kjeldahl nitrogen.

While the mechanism by which the nitrogen fixing ability of nitrogen-fixing bacteria is enhanced by culturing in nitrogen limited medium with addition of an organic acid or its salt is not fully understood, the following is possibly suggested. Nitrogen-fixing bacteria sense 2-oxoglutaric acid/L-glutamine ratios in cells, with the high ratio enhancing nitrogen fixing ability and the low ratio reducing nitrogen fixing ability. Taking up an organic acid in the medium to produce 2-oxoglutaric acid allows nitrogen fixing ability to be enhanced due to a high ratio of 2-oxoglutaric acid/L-glutamine maintained in the cells. The concentration of the organic acid or its salt in the nitrogen limited medium is 0.5 g/L to 100 g/L, and from the viewpoint of maximizing nitrogen fixing ability, it may be 1 g/L or higher, 2.5 g/L or higher, 5 g/L or higher, 6 g/L or higher or 7 g/L or higher, and 15 g/L or lower, 12 g/L or lower, 10 g/L or lower, 9 g/L or lower or 8 g/L or lower. The organic acid salt used may be any salt, with examples including potassium salts, sodium salts and magnesium salts. Organic acids to be added to the nitrogen limited medium may be organic acids in the TCA cycle such as oxaloacetic acid, citric acid, isocitric acid, 2-oxoglutaric acid, succinic acid, fumaric acid and malic acid, as well as their salts.

The carbon source used may be a nitrogen limited medium with addition of a combination of a saccharide such as glucose with the organic acid or its salt. As an example, the amount of the carbon source in the nitrogen limited medium may be 0.5 g/L to 300 g/L and preferably 5 g/L to 100 g/L, in terms of glucose equivalent. The mixing ratio of the saccharide such as glucose to the organic acid or its salt may be selected as appropriate for the experimental conditions, but as an example, the weight ratio of glucose and the citric acid or its salt may be 1:5 to 5:1, preferably 1:4 to 4:1, more preferably 1:3 to 3:1 and even more preferably 1:2 to 2:1. Using a sugar such as glucose at twice the amount of the citric acid or its salt can increase the amount of glutamic acid produced. As an example, when adding glucose and citric acid or its salt to the nitrogen limited medium, the ranges may be 0.5 g/L to 100 g/L for glucose and 0.5 g/L to 50 g/L for citric acid or its salt. The glucose and the citric acid or its salt may each be used at 2.5 g/L or higher, 5 g/L or higher, 6 g/L or higher or 7 g/L or higher, and 15 g/L or lower, 12 g/L or lower, 10 g/L or lower, 9 g/L or lower or 8 g/L or lower, to obtain the mixing ratio mentioned above. From the viewpoint of using nitrogen-fixing bacteria with a transferred citrate transporter gene, the medium used may contain a saccharide such as glucose at twice the amount of 5 to 10 g/L of citric acid or its salt.

The carbon source may be a saccharide such as a monosaccharide, disaccharide, sugar alcohol or polysaccharide, or an organic acid. Such a saccharide may be glucose, mannose, galactose, fructose, xylose, sucrose, maltose, cellobiose, trehalose, mannitol, inositol, sorbitol, glycerol or a starch hydrolysate, but usually glucose. The organic acid used may be any organic acid that can be utilized by bacteria. Examples of organic acids for use as a carbon source include organic acids in the TCA cycle, such as oxaloacetic acid, citric acid, isocitric acid, 2-oxoglutaric acid, succinic acid, fumaric acid and malic acid. The concentration of the carbon source may be set to 0.5 g/L to 300 g/L in terms of glucose equivalent, and from the viewpoint of maximizing nitrogen fixing ability, it is preferably 10 g/L or higher, more preferably 15 g/L or higher and even more preferably 22.5 g/L or higher. The organic acid such as citric acid added to the nitrogen limited medium of the invention is counted as total carbon source, with the concentration of the organic acid such as citric acid and another carbon source such as glucose.

When culturing nitrogen-fixing bacteria, the presence of nitrogen, especially inorganic nitrogen, strongly suppresses the nitrogen fixing reaction. At the initial stage of culturing, a certain level of nitrogen is necessary for growth of the nitrogen-fixing bacteria and production of nitrogenases. Therefore, while a nitrogen source is added to nitrogen limited medium for the initial stage of nitrogen-fixing bacteria, the produced L-glutamic acid or its salt are derived from fixed nitrogen molecules in the atmosphere since nitrogen fixation commences after the depletion of the added nitrogen source. For culturing using the nitrogen limited medium of the invention, therefore, usually no additional nitrogen source will be added other than gaseous nitrogen, such as nitrogen molecules in the air.

The nitrogen limited medium used may also be synthetic minimal medium containing components required depending on the type of nitrogen-fixing bacteria. From the viewpoint of culturing Enterobacteriaceae microorganisms, such components may include inorganic salts, buffer substances and vitamins. Inorganic salts to be added include inorganic salts such as phosphates, sodium salts, magnesium salts, calcium salts, iron salts, manganese salts and molybdenum salts, and more specifically sodium chloride, magnesium sulfate, calcium chloride and molybdic acid salts, such as Na₂ MoO₄·2H₂O. Buffer substances include potassium dihydrogen phosphate and dipotassium hydrogen phosphate. Vitamins include*p*-aminobenzoic acid and biotin. The pH of the nitrogen limited medium may be selected as desired within a range that does not inhibit growth of the nitrogen-fixing bacteria, and it may be adjusted to pH 6.8 to 7.5, for example.

### [Nitrogen-fixing bacteria]

The nitrogen-fixing bacteria to be used for the invention may be microorganisms belonging to Enterobacteriaceae. Examples of microorganisms belonging to Enterobacteriaceae include microorganisms selected from the group consisting of *Klebsiella, Rahnella, Raoultella* and *Kosakonia.* Preferred examples of such microorganisms are *Klebsiella oxytoca, Klebsiella michiganensis, Klebsiella grimontii, Klebsiella pasteurii, Klebsiella pneumoniae, Klebsiella variicola, Klebsiella indica, Rahnella aquatilis, Raoultella terrigena, Raoultella ornithinolytica and Raoultella planticola.* More specifically, *Klebsiella oxytoca,* a nitrogen-fixing bacteria isolated from rice roots, and especially NG13 (Accession No.: NITE BP-03721, deposit number: NITE ABP-03721, date of receipt: July 21, 2023) may be used. NG13 has been deposited at the NITE Patent Microorganisms Depositary of the National Institute of Technology and Evaluation (independent administrative institution). Microorganisms such as *Escherichia coli,* with an introduced gene group associated with nitrogen fixiation, may also be used as the nitrogen-fixing bacteria. Microorganisms belonging to *Xanthobacter* and *Azorhizobium* may also be used as nitrogen-fixing bacteria other than Enterobacteriaceae. Particularly preferred among these microorganisms are microorganisms belonging to *Klebsiella, Rahnella* and *Raoultella.* These microorganisms are able to achieve particularly high production of L-glutamic acid by introducing the citrate synthase gene and citrate transporter gene.

The nitrogen-fixing bacteria of the invention may also have a gene transferred so as to enhance production of L-glutamic acid. Nitrogen-fixing bacteria having a gene transferred so as to enhance production of L-glutamic acid are "nitrogen-fixing bacteria" according to the invention. An example of a transferred gene is a gene coding for a protein selected from among citrate synthase, 2-methylcitrate synthase and citrate transporter. Preferred is introduction of both the citrate synthase gene and citrate transporter gene, or introduction of both the 2-methylcitrate synthase gene and citrate transporter gene. The citrate synthase, 2-methylcitrate synthase and citrate transporter are introduced into nitrogen-fixing bacteria for the purpose of increasing intracellular citric acid concentration. Citrate transporter functions independently from citrate synthase and 2-methylcitrate synthase. This also increases production of 2-oxoglutaric acid as the intracellular concentration of citric acid increases, thus allowing ammonia fixed from nitrogen molecules in the atmosphere to be converted to L-glutamic acid or its salt via the nitrogen assimilation pathway, and resulting in production of L-glutamic acid or its salt.

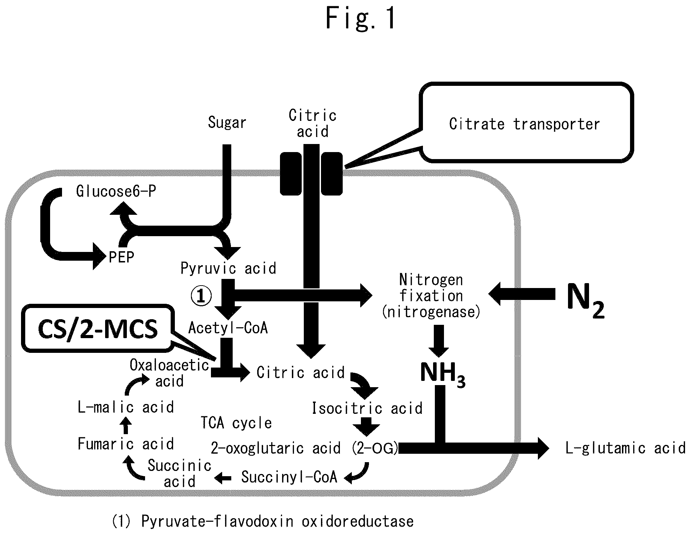

Citrate synthase (CS) is an enzyme that is conserved in almost all organisms and performs a central role in the sugar oxidation process, contributing to the initial stage of the TCA cycle. Specifically, the enzyme catalyzes a reaction which produces citric acid and CoA from acetyl-CoA, converted from pyruvic acid generated in the glycolytic pathway, oxaloacetic acid and water (Fig. 1). According to the invention, the CS gene transferred into nitrogen-fixing bacteria may be selected from that of any bacteria. Bacterial CS includes both "small" CS conserved in Gram-positive bacteria and "large" CS conserved in Gram-negative bacteria. The activity of "small" CS is not inhibited by NADH, but the activity of "large" CS is inhibited by NADH (NPL 3: Weitzman, Adv. Microb. Physiol. 22 185-244 1981). The CS gene transferred into the nitrogen-fixing bacteria is preferably a CS gene acquired from Gram-positive bacteria. For example, it may be a CS gene acquired from *Arthrobacter* bacteria, *Corynebacterium* bacteria, *Bacillus* bacteria, *Mycobacterium* bacteria or *Streptomyces* bacteria. The CS gene of *Corynebacterium glutamicum* may be used, as a specific example. For example, the CS gene of *Corynebacterium glutamicum* ATCC13869 (SEQ ID NO: 3, amino acid sequence: SEQ ID NO: 30) may be used. The activity of CS of some Gram-negative bacteria are not inhibited by NADH, and their CS genes are also preferred as CS genes for being transferred into nitrogen-fixing bacteria. Examples include CS genes acquired from *Acetobacter* bacteria, *Halobacterium* bacteria, *Thermus* bacteria and *Thiobacillus* bacteria. The CS gene of *Thermus aquaticus,* in particular may be used. A CS gene in the host bacteria may also be used. For example, CS of *Klebsiella oxytoca* NG13 is encoded in the nucleotide sequence listed as SEQ ID NO: 1 (amino acid sequence: SEQ ID NO: 28). Transfer of the CS gene into nitrogen-fixing bacteria can increase the citric acid concentration in cells. The nitrogen-fixing bacteria into which the citrate synthase gene has been transferred may be cultured in citric acid-containing nitrogen limited medium to produce L-glutamic acid or its salt.

2-Methylcitrate synthase (2-MCS) is an enzyme conserved in a large number of bacteria, catalyzing reaction which produces 2-methylcitric acid and CoA from propionyl-CoA, oxaloacetic acid and water. On the other hand, 2-MCS also catalyzes the same reaction as CS, as secondary activity. 2-MCS is also often referred to simply as methylcitrate synthase (MCS). According to the invention, the 2-MCS gene transferred into nitrogen-fixing bacteria may be a 2-MCS gene derived from any bacteria so long as the 2-MCS catalyzes the same reaction as CS. For example, the 2-MCS genes of *E. coli* and *Corynebacterium glutamicum* may be used. As more specific examples, the 2-MCS gene derived from *E. coli* W3110 (SEQ ID NO: 5, amino acid sequence: SEQ ID NO: 32) or the 2-MCS gene of *Corynebacterium glutamicum* ATCC13869 (SEQ ID NO: 4, amino acid sequence: SEQ ID NO: 31) may be used. The 2-MCS genes of *Citrobacter* bacteria, *Serratia* bacteria, *Stenotrophomonas* bacteria, *Chromobacterium* bacteria, *Ralstonia* bacteria, *Cupriavidus* bacteria, *Burkholderia* bacteria, *Achromobacter* bacteria and *Herbaspirillum* bacteria, which exhibit at least 73% homology with the amino acid sequence of *E. coli* 2-MCS, may also be used. There may be used the 2-MCS genes of *Corynebacterium comes, Corynebacterium humireducens, Corynebacterium marinum* and *Corynebacterium testudinoris,* as well as *Brevibacterium flavum.* A 2-MCS gene in the host bacteria may also be used. Transfer of the 2-methylcitrate synthase gene into nitrogen-fixing bacteria can increase the citric acid concentration in cells. The nitrogen-fixing bacteria into which the 2-MCS gene has been transferred may be cultured in citric acid-containing nitrogen limited medium to produce L-glutamic acid or its salt.

Citrate transporter is a transporter that takes up citric acid into bacteria from extracellular environment. Bacterial citrate transporters are classified into 5 types, specifically the classification (TC) system numbers of 2.A.1.6, 2.A.11, 2.A.24, 2.A.47 and 2.A.80 (NPL 4: Saier, Microbiol. Mol. Biol. Rev. 64 354-411 2000, NPL 5: Soares-Silva et al., FEMS Microbiol. Letter. 367 1-15 2020). Transporter TC No.2.A.1.6 is a citric acid:H⁺ symporter, typically CitH of *Klebsiella pneumoniae.* This transporter is conserved in numerous bacteria. Transporter TC No.2.A.11 includes citric acid·divalent metal ion:H⁺ symporter represented by CitM in *Bacillus subtilis,* citric acid·divalent metal ion:H⁺ symporter represented by CitH in *Bacillus subtilis,* citric acid:H⁺ symporter represented by YRAO in *Bacillus subtilis,* and citric acid·divalent metal ion transporter represented by CitH in *Corynebacterium glutamicum.* Transporter TC No.2.A.24 includes citric acid:Na⁺ symporter represented by CitS of *Klebsiella pneumoniae,* citric acid:Na⁺ symporter represented by MleP of *Lactococcus lactis,* electrically neutral citric acid:H⁺ symporter represented by CimH of *Bacillus subtilis,* electrogenic citric acid:L-lactic acid exchanger represented by CitP of *Lactococcus lactis,* and citric acid:lactic acid antiporter represented by CitP of *Leuconostoc mesenteroides.* Transporter TC No.2.A.47 is citric acid: succinic acid antiporter represented by CitT of *E. coli.* Transporter TC No.2.A.80 is three-molecule complex citrate transporter represented by TctCBA of *Corynebacterium glutamicum.* According to the invention, the citrate transporter gene transferred into nitrogen-fixing bacteria may be a gene derived from any bacteria which codes for any of the aforementioned citrate transporters. A citrate transporter gene in the host bacteria may also be used. It is especially preferred to transfer the citric acid:Na⁺ symporter gene and the citric acid:H⁺ symporter gene which are conserved in *Klebsiella oxytoca.* Since *Klebsiella oxytoca* conserves a homologous gene to the citric acid:acetic acid exchanger gene which is conserved in *Klebsiella pneumoniae* (*citW*) (NPL 6: Kaestner et al., Arch. Microbiol. 177 500-506 2002), the citric acid:acetic acid exchanger gene conserved in *Klebsiella oxytoca* can also be transferred. For example, citric acid·Na⁺ symporter of *Klebsiella oxytoca* NG13 is encoded in the nucleotide sequence listed as SEQ ID NO: 2 (amino acid sequence: SEQ ID NO: 29). The nitrogen-fixing bacteria into which the citrate transporter gene has been transferred may be cultured in citric acid-containing nitrogen limited medium to increase the citric acid concentration in the bacteria and to produce L-glutamic acid or its salt.

Nitrogen-fixing bacteria usually have lower nitrogen fixing ability when the genes of their metabolic pathways are modified. It is therefore necessary to use culture medium allowing a high level of nitrogen fixing ability to be exhibited even with nitrogen-fixing bacteria having genetically modified metabolic pathways. Nitrogen limited medium containing an organic acid or its salt can enhance nitrogen fixing ability of the modified nitrogen-fixing bacteria and will enable their fermentative production of L-glutamic acid or its salt from nitrogen molecules in the atmosphere.

A gene coding for a protein selected from the group consisting of citrate synthase, 2-methylcitrate synthase and citrate transporter may also comprise the promoter and terminator sequences that control its expression. It is more preferred to use a promoter that enables transient expression. The promoter which enables transient expression may be *trc* promoter, which can be controlled by addition of IPTG. A vector containing a gene comprising the promoter and terminator may be used for gene expression by an established method.

Nitrogen-fixing bacteria with at least one gene selected from the group consisting of citrate synthase, 2-methylcitrate synthase and citrate transporter, to be used for the invention, may also have another gene transferred for the purpose of producing an amino acid other than L-glutamic acid. Amino acids other than L-glutamic acid include essential amino acids such as histidine, isoleucine, leucine, lysine, methionine, phenylalanine, threonine, tryptophan and valine, and amino acids such as arginine, aspartic acid, glutamine, asparagine, alanine, glycine, cysteine, aspartic acid, proline, glutamine, tyrosine and γ-aminobutyric acid, and will generally be L-form amino acids. An amino acid other than L-glutamic acid may be produced by selecting an appropriate transgene based on known technology in the fermentation of amino acids. As an example, L-alanine and L-valine can be produced by transferring the glutamate-pyruvate aminotransferase gene (SEQ ID NO: 33, amino acid sequence: SEQ ID NO: 37) and the L-alanine transporter gene (SEQ ID NO: 34, amino acid sequence: SEQ ID NO: 38). As another example, it is possible that L-aspartic acid can be produced by transferring the glutamate-oxaloacetate aminotransferase gene (SEQ ID NO: 35, amino acid sequence: SEQ ID NO: 39) (NPL 7: Son HF et al., PLoS ONE. 11(6): e0158402). As yet another example, it is possible that γ-aminobutyric acid can be produced by transferring the glutamate decarboxylase gene (SEQ ID NO: 36, amino acid sequence: SEQ ID NO: 40) (NPL 8: Choi, J. W. et al., Microb Cell Fact 14, 21 2015). According to a different aspect, therefore, the present invention may relate to nitrogen-fixing bacteria having another transferred gene other than at least one gene selected from the group consisting of citrate synthase, 2-methylcitrate synthase and citrate transporter, for the purpose of producing an amino acid other than L-glutamic acid. According to yet another aspect, the invention may relate to a method for fermentatively producing an amino acid other than L-glutamic acid, such as L-alanine and/or L-valine, containing a step of culturing the nitrogen-fixing bacteria in nitrogen limited medium containing an organic acid or its salt. The nitrogen limited medium used in the method for fermentatively producing an amino acid other than L-glutamic acid, such as L-alanine and/or L-valine, may be the same medium as used in the method for fermentatively producing L-glutamic acid.

Glutamate-pyruvate aminotransferase is an enzyme that catalyzes a reaction producing L-alanine and 2-oxoglutaric acid from L-glutamic acid and pyruvic acid, and thus contributes to biosynthesis of L-alanine. The glutamate-pyruvate aminotransferase gene may be derived from any bacteria, and transfer of the glutamate-pyruvate aminotransferase gene into nitrogen-fixing bacteria causes transfer of the amino residue of L-glutamic acid derived from nitrogen molecules in the atmosphere to pyruvic acid, thereby producing L-alanine or its salt.

L-alanine transporter functions to transport L-alanine out of the cell. The L-alanine transporter gene may be derived from any bacteria, and transfer of the L-alanine transporter gene into nitrogen-fixing bacteria together with the glutamate-pyruvate aminotransferase gene is expected to enhance extracellular transport of the produced L-alanine, and increase productivity of L-alanine or its salt.

Glutamate-oxaloacetate aminotransferase is an enzyme that catalyzes a reaction producing L-aspartic acid and 2-oxoglutaric acid from L-glutamic acid and oxaloacetic acid, and thus contributes to biosynthesis of L-aspartic acid. The glutamate-oxaloacetate aminotransferase gene may be derived from any bacteria, and transfer of the glutamate-oxaloacetate aminotransferase gene into nitrogen-fixing bacteria is expected to cause transfer of the amino residue of L-glutamic acid derived from nitrogen molecules in the atmosphere to oxaloacetic acid, thereby producing L-aspartic acid or its salt.

Glutamate decarboxylase is an enzyme that catalyzes a reaction producing γ-aminobutyric acid from L-glutamic acid, and thus contributes to biosynthesis of γ-aminobutyric acid. The glutamate decarboxylase gene may be derived from any bacteria, and transfer of the glutamate decarboxylase gene into nitrogen-fixing bacteria is expected to cause decarboxylation of the L-glutamic acid derived from nitrogen molecules in the atmosphere, thereby producing γ-aminobutyric acid or its salt.

The gene transferred according to the invention may have at least 75%, at least 80%, at least 85%, at least 90%, at least 95%, at least 97%, at least 98% or at least 99% sequence homology, or sequence identity, with the sequence of each original gene, and with the same or similar enzyme activity when expressed. Homology can be determined using the NCBI BLASTn program accessing the NCBI registered database. Specifically, a transferred gene may have at least 75%, at least 80%, at least 85%, at least 90%, at least 95%, at least 97%, at least 98% or at least 99% sequence identity with the sequences of the CS genes of SEQ ID NO: 1 and 3, and having citrate synthase activity when expressed. As another example, a transferred gene may have at least 75%, at least 80%, at least 85%, at least 90%, at least 95%, at least 97%, at least 98% or at least 99% sequence identity with the sequences of the citric acid·Na⁺ symporter gene of SEQ ID NO: 2, and having citric acid· Na⁺ symport activity when expressed. According to yet a different aspect, a transferred gene may have at least 75%, at least 80%, at least 85%, at least 90%, at least 95%, at least 97%, at least 98% or at least 99% sequence identity with the sequences of the 2-methylcitrate synthase genes of SEQ ID NO: 4 and 5, and having 2-methylcitrate synthase activity when expressed.

The gene transferred according to the invention may be a gene coding for an amino acid sequence having at least 75%, at least 80%, at least 85%, at least 90%, at least 95%, at least 97%, at least 98% or at least 99% sequence homology, or sequence identity, with the amino acid sequence of each original gene, and with the same or similar enzyme activity when expressed. Homology can be determined using the NCBI BLASTp program accessing the NCBI registered database. Specifically, a transferred gene codes for an amino acid sequence having at least 75%, at least 80%, at least 85%, at least 90%, at least 95%, at least 97%, at least 98% or at least 99% sequence identity with the CS amino acid sequences of SEQ ID NO: 28 and 30, and having citrate synthase activity. As another example, a transferred gene codes for an amino acid sequence having at least 75%, at least 80%, at least 85%, at least 90%, at least 95%, at least 97%, at least 98% or at least 99% sequence identity with the amino acid sequence of citric acid·Na⁺ symporter of SEQ ID NO: 29, and having citric acid· Na⁺ symport activity. According to yet a different aspect, a transferred gene codes for an amino acid sequence having at least 75%, at least 80%, at least 85%, at least 90%, at least 95%, at least 97%, at least 98% or at least 99% sequence identity with the 2-methylcitrate synthase amino acid sequences of SEQ ID NO: 31 and 32, and having 2-methylcitrate synthase activity.

### [Method for fermentatively producing L-glutamic acid]

The method for fermentatively producing L-glutamic acid or its salt from nitrogen molecules in the atmosphere, according to another aspect of the invention, comprises a step of culturing nitrogen-fixing bacteria having a gene transferred so as to enhance production of L-glutamic acid, in a nitrogen limited medium containing an organic acid, i.e. a nitrogen limited medium of the invention. By culturing nitrogen-fixing bacteria having a gene transferred so as to enhance production of L-glutamic acid in the nitrogen limited medium containing an organic acid, it is possible to enhance the nitrogen fixing ability and the fermentation of L-glutamic acid or its salt from nitrogen molecules in the atmosphere.

The culturing conditions for the invention may be appropriately selected depending on the nitrogen-fixing bacteria used, alternatively to static culture under air in a non-hermetic vessel. For example, in order to increase the anaerobic level, nitrogen limited medium and air may be placed in the hermetic vessel so that the dissolved oxygen present at the start of culture is immediately consumed to reinforce the anaerobic state. Culturing may also be carried out in a completely anaerobic state by placing nitrogen gas and nitrogen limited medium in the hermetic vessel. Alternatively, the contact area between the culture and air may be enlarged in static culture under air in anon-hermetic vessel, for more aerobic culturing. From the viewpoint of culturing of Enterobacteriaceae microorganisms, the culturing temperature may be 20°C to 37°C and preferably 20 to 30°C.

In the culturing step of the invention, a nitrogen source is necessary in culture medium under nitrogen limitation for initial growth of the nitrogen-fixing bacteria. The nitrogen source in the culture medium under nitrogen limitation is consumed by cells during initial growth, and then when the nitrogen concentration in the culture medium falls below a certain concentration, the nitrogen-fixing bacteria begin to fix nitrogen in the atmosphere. During this time, the nitrogen-fixing bacteria, which have a gene transferred so as to enhance L-glutamic acid production, exhibit increased intracellular citric acid concentration, and the fixed ammonia is converted to L-glutamic acid or its salt through the nitrogen assimilation pathway, thereby producing L-glutamic acid or its salt.

All of the publications mentioned throughout the present specification are incorporated herein in their entirety by reference.

The Examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

### EXAMPLES

### Example 1: Examining culturing conditions

It has conventionally been thought that NG13 (Accession No.: NITE BP-03721, deposit number: NITE ABP-03721, date of receipt: July 21, 2023) exhibits nitrogen fixing ability only in anaerobic culturing. Anaerobic culturing is a complex culturing method with many procedures, including sealing up the culture vessel and replacing the air layer with nitrogen gas or a mixture of nitrogen gas and argon gas. Therefore, a completely novel culturing method was established in which an non-hermetic vessel is used, and nitrogen molecules in the atmosphere are freely incorporated to exhibit nitrogen fixing ability. The culture medium used was nitrogen limited medium comprising nitrogen-free medium with addition of 100 mg/L yeast extract.

After placing 5 mL of nitrogen limited medium (KD culture medium containing 7.5 g/L glucose alone, see Table 1) in a φ18 × 180 mm test tube and inoculating NG13 at 10⁷ cells, the static culture was carried out at 25°C. The top of the test tube was covered with an aluminum cap, allowing air outside the test tube to freely enter into the test tube. The cell density in the culture was measured, and the nitrogen fixing ability exhibited in the culture was also measured by the acetylene reduction method (described below). NG13 initiated its growth in a short lag time after the start of culturing, and exhibited nitrogen fixing ability of 0.63 µmol/4-h/tube at 24 hours after the start of culturing (see Table 2). The level of nitrogen fixing ability was the same as the nitrogen fixing ability exhibited with anaerobic culturing when the top of the test tube was sealed with a butyl rubber and the air layer was filled with nitrogen gas. The maximum nitrogen fixing ability was equivalent even when the glucose in the KD culture medium was replaced with an equal amount of fructose or sucrose. A completely novel, convenient culturing method was thus established for causing NG13 to exhibit nitrogen fixing ability.

The procedure for the acetylene reduction method was as follows. First, the top of the test tube was sealed with a butyl rubber. The air layer was air. After then replacing 15% of the air layer with acetylene, static culture was carried out for 4 hours at 25°C. After culturing, 0.5 mL of the air layer was extracted with a syringe and the amount of ethylene was measured with a gas chromatograph, converting it to production of ethylene per test tube, as the value for the nitrogen fixing ability.

Next, in order to search for nitrogen limited media that enhance the nitrogen fixing ability of NG13, nitrogen limited media containing various organic acids in addition to glucose were prepared as shown in Table 1, and NG13 was statically cultured in each medium for 24 hours at 25°C, and the nitrogen fixing ability was measured. The results are shown in Table 2.

**[Table 1]**

| Table 1 (Units: mg/L) | | | | | | | | |
|---|---|---|---|---|---|---|---|---|
| Medium name | KD | KDG | KDC | KDM | KDS | KDF | KDO | KDP |
| K₂ HPO₄ | 800 | 800 | 800 | 800 | 800 | 800 | 800 | 800 |
| KH₂ PO₄ | 200 | 200 | 200 | 200 | 200 | 200 | 200 | 200 |
| NaCl | 100 | 100 | 100 | 100 | 100 | 100 | 100 | 100 |
| NaFe-EDTA | 1 | 1 | 1 | 1 | 1 | 1 | 1 | 1 |
| Na₂ MoO₄ • 2H₂ O | 1 | 1 | 1 | 1 | 1 | 1 | 1 | 1 |
| MgSO₄ | 98 | 98 | 98 | 98 | 98 | 98 | 98 | 98 |
| CaCl₂ • 2H₂ O | 60 | 60 | 60 | 60 | 60 | 60 | 60 | 60 |
| Glucose | 7500 | 1500 0 | 7500 | 7500 | 7500 | 7500 | 7500 | 7500 |
| Sodium citrate | - | - | 7500 | | - | - | - | - |
| Sodium malate | | | | 7500 | | | | |
| Sodium succinate | - | - | - | | 7500 | - | - | - |
| Sodium fumarate | - | - | - | | - | 7500 | - | - |
| Sodium 2-oxoglutarat e | - | - | - | | - | - | 7500 | - |
| Sodium pyruvate | - | - | - | | - | - | - | 7500 |
| Bacto Yeast Extract | 100 | 100 | 100 | 100 | 100 | 100 | 100 | 100 |
| Biotin | 0.005 | 0.005 | 0.005 | 0.005 | 0.005 | 0.005 | 0.005 | 0.005 |
| *p*-Aminobenzoic acid | 0.01 | 0.01 | 0.01 | 0.01 | 0.01 | 0.01 | 0.01 | 0.01 |

### [Table 2]

**Table 2**

| Medium | Maximum nitrogen fi xing ability (*µ*mol/4-h/tube) | Final bacteria count (10⁹ cells/tub e) | Enhancement ratio for m aximum nitrogen fixing a bility compared to KD me dium culturing |
|---|---|---|---|
| KD | 0.63 | 1.17 | - |
| KDG | 0.70 | 1.10 | 1.1 |
| KDC | 1.66 | 1.52 | 2.6 |
| KDM | 1.14 | 1.47 | 1.8 |
| KDS | 1.50 | 1.44 | 2.4 |
| KDF | 0.95 | N.T. | 1.5 |
| KDO | 1.05 | 1.47 | 1.7 |
| KDP | 0.69 | N.T. | 1.1 |

While the nitrogen fixing ability was not enhanced in KDD medium with 7.5 g/L glucose added to KD medium to double the glucose amount (15 g/L), or KDP medium with sodium pyruvate (7.5 g/L) added to KD medium, nitrogen fixing ability was enhanced in other media with sodium salts of different organic acids (7.5 g/L) added to KD medium. The effect was greatest for combination of sodium citrate and glucose (KDC medium) (2.6-fold enhancement). However, no accumulation of L-glutamic acid was found in all the tested medium.

In order to search for optimal sodium citrate concentrations that enhance nitrogen fixing ability of NG13 and NG13-pCCS in KDC medium, nitrogen limited media containing various concentrations of sodium citrate with 7.5 g/L glucose were prepared as shown in Table 3, and both strains were statically cultured in each culture medium for 24 hours at 25°C, and the nitrogen fixing ability was measured by the acetylene reduction method. The results are shown in Table 3.

### [Table 3]

**Table 3**

| Strain | Sodium citrate (g/L) | Maximum nitrogen fixing ability (µmol/4-h/tube) | L-Glutamic acid production in Day1 (mg/L) |
|---|---|---|---|
| NG13 | 0 (KD medium) | 0.45 | N.D. |
| | 1 | 0.45 | N.D. |
| | 2.5 | 0.67 | N.D. |
| | 5 | 0.86 | N.D. |
| | 7.5 (KDC medium) | 1.12 | N.D. |
| | 15 | 1.33 | N.D. |
| NG13-pCCS | 0 (KD medium) | 0.20 | 11.0 |
| | 1 | 0.22 | 17.1 |
| | 2.5 | 0.36 | 27.2 |
| | 5 | 0.46 | 32.6 |
| | 7.5 (KDC medium) | 0.52 | 33.2 |
| | 15 | 0.47 | 31.7 |

A sodium citrate concentration of 2.5 g/L to 15 g/L in the culture medium enhanced maximum nitrogen fixing ability using NG13, compared to 0 g/L of sodium citrate (KD medium). A sodium citrate concentration of 1 g/L to 15 g/L in the culture medium enhanced maximum nitrogen fixing ability using NG13-pCCS, compared to 0 g/L of sodium citrate (KD medium), with the highest ability being exhibited with 7.5 g/L (KDC medium).

### Example 2 Preparation of NG13 strains with a transferred gene of citrate synthase, 2-methylcitrate synthase or citric acid:Na⁺ symporter

### (1) Construction of plasmids

Genomic DNA was extracted from NG13, *Corynebacterium glutamicum* ATCC13869 (hereunder, "ATCC13869") and *E. coli* W3110 (hereunder, "W3110"), using a Wizard Genomic DNA Purification Kit (Promega) or InstaGene (Bio-Rad). Gene fragments coding for citrate synthase (CS, ATCC13869: BBD29_04605) and 2-methylcitrate synthase (2-MCS, ATCC13869: BBD29_03720 and W3110: PrpC) were amprified by the PCR using the primers listed in Table 4 and the genomic DNA as template with the combinations listed in Table 5. The citric acid:Na⁺ symporter gene of NG13 was cloned based on genomic information of *Klebsiella oxytoca* M5a1 to acquire sequence information, and then gene fragments were amprified by PCR. The primers were designed so that an approximately 20 bp sequence homologous to the vector was added to both ends, and so that the introduced gene was expressed using the expression promoter and ribosome binding site (RBS) in the vector (Table 4). The fragment of the vector was also prepared by PCR. The sequences and combinations of primers used were as shown below. The target gene fragments and vector fragments were ligated by the Gibson Assembly. A 10 µL portion of reaction mixture containing both fragments was used to transform *E. coli* DH5α, and a drug selection marker in the plasmids was used to screen the colonies. The plasmids were extracted from the appearing colonies and subjected to sequence analysis to confirm that the plasmids were correctly designed (Table 5).

**[Table 4]**

| Table 4. Primer 5' →3' sequences used are shown. Underlines indicate sequences used in Gibson Assembly | | |
|---|---|---|
| Primer Name | Sequence | SEQ ID No. |
| 1 | AAGGATCTAGGTGAAGATCC | 6 |
| 2 | GGTTATTGTCTCATGAGCGG | 7 |
| 3 | CCGCTCATGAGACAATAACCAATTCTCATGTTTGACAG | 8 |
| 4 | GGATCTTCACCTAGATCCTTATTCAGGTCGAGGTGGCCCG | 9 |
| 5 | GGTTTATTCCTCCTTATTTAATCG | 10 |
| 6 | AATTCGAAGCTTACGTAGAAC | 11 |
| 7 | CTCCCGTTCTGGATAATG | 12 |
| 8 | CGGGTACCGAGGTGGAATTCTTAGCGCTCCTCGCGAGG | 13 |
| 9 | GAATTCGAGCTCGGTACC | 14 |
| 10 | CGGGTACCGAGCTCGAATTCTTAACGCTTTTCAATGGG | 15 |
| 11 | CGGGTACCGAGCTCGAATTCTTACTGGCGCTTATCCAG | 16 |
| 12 | CATGGACTCGTCTACTAG | 17 |
| 13 | AACATTATCCAGAACGGGAGTCCTAATGCAGGAGTCGC | 18 |
| 14 | CGATTAAATAAGGAGGAATAAACCATGTTTGAAAGGGATATC | 19 |
| 15 | GGGTGTTGGCGGGTGTCGGGCGCTCACTGCCCGCTTTC | 20 |
| 16 | CCCGACACCCGCCAACACCC | 21 |
| 17 | CGATTAAATAAGGAGGAATAAACCATGTCCAGCGCCACAACC | 22 |
| 18 | CGATTAAATAAGGAGGAATAAACCATGAGCGACACAACGATC | 23 |
| 19 | CGATTAAATAAGGAGGAATAAACCATGACTAATATGAGCCAGGC | 24 |
| 20 | CGATTAAATAAGGAGGAATAAACCATGTCTGATGCTAAAGCA | 25 |
| 21 | GTTCTACGTAAGCTTCGAATTTTAGCGGGCGATATCGTT | 26 |
| 22 | CGCTAGTAGACGAGTCCATGTTACATCATCATGCCGAAC | 27 |
| 23 | CGATTAAATAAGGAGGAATAAACCATGGCTGAATTCAGTCCTG | 41 |
| 24 | GTGCTCCTCGAAAATGAAGTCGTTATTCGTTGCTTTCGCTG | 42 |
| 25 | CGACTTCATTTTCGAGGAG | 43 |
| 26 | CGCTAGTAGACGAGTCCATGTCAACTCAGGCTTTGGCC | 44 |
| 27 | CTAGTAGACGAGTCCATGTTATTCGTTGCTTTCGCTGG | 45 |

**[Table 5]**

| Table 5. List of information relating to name of created plasmids and proteins encoded by expressed genes, and information relating to templates and primers used for plasmid creation | | | | | | | | | | | |
|---|---|---|---|---|---|---|---|---|---|---|---|
| Plasmid name | Expressed gene (source) | ori | Drug resistance gene | Fragment 1 | | Fragment 2 | | Fragment 3 | | Fragment4 | |
| | | | | Template | Primers | Template | Primers | Template | | Template | Primers |
| pCCS | Citrate synthase (C.glutamicum ATCC13869) | pSC101 | Tc | Genomic DNA (ATCC13869) | 14, 8 | pMW118 | 9, 2 | 707-FLPe (Tc^{R}) | 3, 4 | pTrcHis2-TOPO/lacZ (lacI^{q}, Ptrc, lacO) | 16. 5 |
| pCMCS | 2-Methylcitrate synthase (C.glutamicum ATCC13869) | pSC101 | Tc | Genomic DNA (ATCC13869) | 17, 10 | pMW118 | 9, 2 | 707-FLPe (Tc^{R}) | 3, 4 | pTrcHis2-TOPO/lacZ (lacI^{q}, Ptrc, lacO) | 16, 5 |
| pEMCS | 2-Methylcitrate synthase (E.coli W3110) | pSC101 | Tc | Genomic DNA (W3110) | 18, 11 | pMW118 | 9, 2 | 707-FLPe (Tc^{R}) | 3,4 | pTrcHis2-TOPO/lacZ (lacI^{q}, Ptrc, lacO) | 16, 5 |
| pKCS | Citrate synthase (K. oxytoca NG13) | pBR322 | Tc | Genomic DNA (NG13) | 20, 21 | pTrcHis2-TOPO/lacZ | 6, 2 | 707-FLPe (Tc^{R}) | 3,15 | pTrcHis2-TOPO/lacZ (lacI^{q}, Ptrc, lacO) | 1, 5 |
| pKCT | Na*-dependent citrate transporter (K.oxytoca NG13) | pCDF | Spc | Genomic DNA (NG13) | 19, 22 | pCDFDuet-1 (Spc^{R}, lacI) | 12, 13 | pTrcHis2-TOPO/lacZ (Ptrc. lacO) | 7, 5 | - | - |
| pKACE | Glutamate-pyruvate aminotransferase, alanine transporter (K.oxytoca NG13) | p15A | Cm | Genomic DNA (NG13) | 23, 24 | Genomic DNA (NG13) | 25, 26 | pACYCDuet-1 (Cm^{R}, lacI) | 12, 13 | pTrcHis2-TOPO/lacZ (Ptrc, lacO) | 7, 5 |

### (2) Introduction of plasmids into NG13

The NG13 was grown in LB liquid medium (1% Bacto Tryptone, 0.5% Bacto Yeast Extract, 1% NaCl) to approximate OD₆₀₀ = 0.5 and the cells were harvested, and after washing one time each in 40 mL of ice-cooled sterilized water and 40 mL of 10% glycerol, the cells were suspended in 10% glycerol to approximate OD₆₀₀ = 100 and dispensed in 60 µL portions to stock as competent cells. After mixing approximately 200 ng of each plasmid, a GENE PULSER II (BioRad) was used for introduction at 25 kV/cm, 25 µF and 200 Ω. The introduced competent cells were mixed with 1 mL of antibiotic-free LB liquid medium and cultured at 30°C for 1 hour at 300 rpm, after which the mixture was spread onto an LB agar plate containing antibiotics (spectinomycin: 100 µg/mL, tetracycline: 15 µg/mL) and cultured overnight at 30°C to obtain transformants. The plasmids were extracted from the appearing colonies, and restriction enzyme treatment and subsequent agarose gel electrophoresis were carried out to confirm that the target plasmid was properly retained. The target transformants were stored at -80°C as a 25% glycerol suspension.

### Example 3: Fermentative production of L-glutamic acid by NG13 with a transferred gene of 2-methylcitrate synthase

Since the nitrogen fixing ability of NG13 is exhibited under anaerobic conditions, modified strains NG13 having a plasmid pCMCS which expresses the 2-MCS gene of *Corynebacterium glutamicum* (NG13-pCMCS), and NG13 having a plasmid pEMCS which expresses the 2-MCS gene of *E. coli* (NG13-pEMCS), as described in PTL 2 (Japanese Unexamined Patent Publication No. 2009-254323), were constructed and both strains were statically cultured for 4 days at 25°C in KDC medium with addition of IPTG to a final concentration of 0.1 mM, and their L-glutamic acid productivity was examined. The results are shown in Table 6.

### [Table 6]

**Table 6**

| Strain | Cultu re me dium | Maximum nitroge n fixing ability (*µ*mol/4-h/tub e) | Final bacteri a count (10⁹ cells/tu be) | L-glutamic acid concentr ation in Day4 (mg/L) |
|---|---|---|---|---|
| NG13 | KDC | 1.42 | 1.8 | N.D. |
| NG13-pCMCS | KDC | 0.79 | 1.1 | 74.2 |
| NG13-pEMCS | KDC | 0.68 | 1.2 | 52.3 |

L-glutamic acid accumulated in the culture medium with both modified NG13 strains.

### Example 4: Fermentative production of L-glutamic acid by NG13 with a transferred gene of citrate synthase

PTL 1 (Japanese Patent Publication No. 4144131) reports that aerobic culturing of *Klebsiella* bacteria having the CS gene derived from *Corynebacterium* bacteria results in fermentative production of 2.85 g/L L-glutamic acid. NG13 having a plasmid pCCS expressing the CS gene of *Corynebacterium glutamicum* (NG13-pCCS) was constructed in the same manner as disclosed in PTL 1. First, the modified strain was cultured at 250 rpm at 30°C in KDC medium with addition of 10 g/L ammonium chloride and IPTG to a final concentration of 0.1 mM, which was a non-diazotrophic condition, resulting in a final bacteria count of 8 × 10¹⁰ cells/tube at 28 hours after the start of culturing, and accumulating 1.4 g/L L-glutamic acid in the culture medium. Also NG13 having a plasmid pKCS which expresses the CS gene of NG13 itself (NG13-pKCS) was constructed and cultured at 250 rpm at 30°C in KDC medium with addition of 1 g/L ammonium chloride and IPTG to a final concentration of 0.1 mM, resulting in a final bacteria count of 0.5 × 10¹⁰ cells/tube at 18 hours after the start of culturing, and accumulating 20 mg/L L-glutamic acid in the culture medium.

The NG13-pCCS and NG13-pKCS strains were then statically cultured for 5 days at 25°C in KDC medium with addition of IPTG to a final concentration of 0.1 mM, which was a diazotrophic condition, and their L-glutamic acid productivity was examined. The results are shown in Table 7.

### [Table 7]

**Table 7**

| Strain | Cultu re me dium | Maximum nitroge n fixing ability (*µ*mol/4-h/tub e) | Final bacteri a count (10⁹ cells/tu be) | L-glutamic acid concentr ation in Day5 (mg/L) |
|---|---|---|---|---|
| NG13 | KDC | 1.35 | 1.80 | N.D. |
| NG13-pCCS | KDC | 0.52 | 0.97 | 84.5 |
| NG13-pKCS | KDC | 0.32 | 1.24 | 16.5 |

NG13-pCCS accumulated L-glutamic acid in the culture medium, the amount exceeded those by strains having a transferred gene of the 2-MCS as described above. Considering the bacteria counts, it may be concluded that NG13-pCCS produces L-glutamic acid in an equal amount whether the ammonia is taken up from the outside or the ammonia is produced by nitrogen fixing. However, the accumulated amount of L-glutamic acid with NG13-pKCS was less than that of NG13-pCCS.

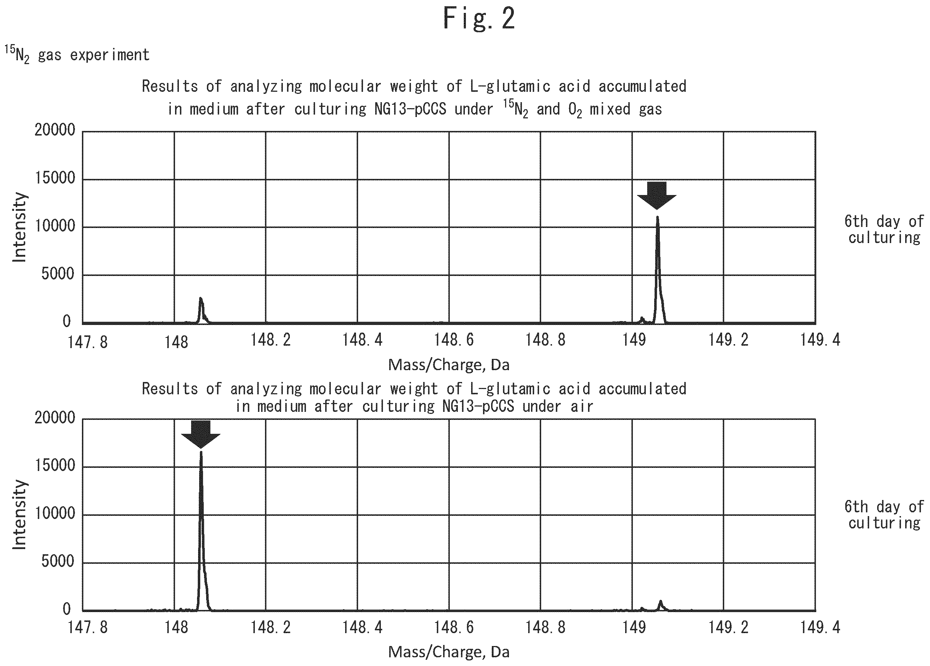

In order to confirm that the nitrogen atom introduced into the produced L-glutamic acid was derived from nitrogen molecules in the atmosphere, NG13-pCCS was cultured in KDC medium lacking Na₂ MoO₄·2H₂O in order to eliminate its nitrogen fixing ability. As a result, absolutely no accumulation of L-glutamic acid was observed in the culture medium even though the final bacteria count in the culture solution was the same as when cultured in normal KDC medium. Moreover, when NG13-pCCS was cultured in KDC medium, with the top of the test tube sealed with a butyl rubber and the air layer filled with a mixed gas of ¹⁵N₂ and O₂ (¹⁵N₂:O₂ = 82:18), L-glutamic acid was accumulated in the culture at 120 mg/L in 6 days of culturing. LC-MS analysis showed that the majority consisted of L-glutamic acid cation ([C₅H₁₀¹⁵NO₄]⁺) with a molecular weight of 149, containing ¹⁵N (Fig. 2). This showed that the nitrogen atom of L-glutamic acid produced in the KDC medium was mostly derived from nitrogen molecules in the atmosphere by nitrogen fixiation.

NG13-pCCS was then statically cultured for 6 days at 25°C in KD medium, KDD medium, KDC medium, KDM medium, KDS medium and KDO medium, and it was examined whether the presence of organic acids other than citric acid in the nitrogen limited medium affected production of L-glutamic acid. The results are shown in Table 8.

### [Table 8]

**Table 8**

| Strain | Cultu re me dium | Maximum nitroge n fixing ability (*µ*mol/4-h/tub e) | Final bacteri a count (10⁹ cells/tu be) | L-glutamic acid concentr ation in Day6 (mg/L) |
|---|---|---|---|---|
| NG13-pCCS | KD | 0.25 | 0.76 | 28.5 |
| NG13-pCCS | KDD | 0.28 | 0.82 | 26.0 |
| NG13-pCCS | KDC | 0.54 | 0.71 | 109.2 |
| NG13-pCCS | KDM | 0.46 | 1.02 | 56.1 |
| NG13-pCCS | KDS | 0.56 | 1.16 | 63.6 |
| NG13-pCCS | KDO | 0.39 | 0.88 | 79.3 |

No difference was observed between culturing in KD medium and KDD medium in terms of maximum nitrogen fixing ability, final bacteria count or L-glutamic acid productivity. L-Glutamate production in KDD medium was the least of the six media tested. On the other hand, the maximum nitrogen fixing ability was enhanced and L-glutamic acid productivity was greatly increased when culturing in KDC medium, KDM medium, KDS medium and KDO medium, compared to culturing in KD medium.

### Example 5: Fermentative production of L-glutamic acid by NG13 with a transferred gene of citrate transporter

When NG13 is statically cultured in KDC medium, glucose is assimilated at first, with very little assimilation of citric acid. Citric acid assimilation commences after the glucose has been depleted. NG13 has the citric acid:Na⁺ symporter gene and a gene for a two-component control system (composed of the sensor kinase CitA and the response regulator CitB) that senses the extracellular presence of citric acid, and expression of the citric acid:Na⁺ symporter gene is promoted by phosphorylated CitB (NPL 2: Bott, Mol. Microbiol. 18 533-546 1995). When NG13 is cultured in KDC medium, CitA may sense a high concentration of citric acid in the medium and phosphorylate CitB to promote expression of the citric acid:Na⁺ symporter gene. It is possible that this causes slight assimilation of citric acid even in the presence of glucose. Expression of citric acid:Na⁺ symporter gene, on the other hand, is also promoted by cyclic AMP receptor protein (CRP). This is also thought to be related to assimilation of citric acid after glucose consumption.

NG13 citric acid:Na⁺ symporter gene was therefore cloned and plasmid pKCT was constructed for its overexpression in NG13. NG13 having the plasmid introduced (NG13-pKCT) was statically cultured for 5 days at 25°C in KDC medium, and its L-glutamic acid productivity was examined. The results are shown in Table 9.

### [Table 9]

**Table 9**

| Strain | Cultu re me dium | Maximum nitroge n fixing ability (*µ*mol/4-h/tub e) | Final bacteri a count (10⁹ cells/tu be) | L-glutamic acid concentr ation in Day5 (mg/L) |
|---|---|---|---|---|
| NG13 | KDC | 1.05 | 1.71 | N.D. |
| NG13-pKCT | KDC | 0.85 | 1.75 | 22.4 |

The maximum nitrogen fixing ability and final bacteria count of NG13-pKCT were not significantly different from those of NG13, indicating that the basal production of citric acid:Na⁺ symporter is sufficient for enhancing maximum nitrogen fixing ability in the presence of citric acid. Focusing on accumulation of L-glutamic acid, it is necessary to enhance the production of citric acid:Na⁺ symporter for citric acid uptake to cause accumulation of L-glutamic acid.

### Example 6: Fermentative production of L-glutamic acid by NG13 with cotransferred genes of citrate synthase and citrate transporter

When NG13-pKCT and NG13-pCCS were cultured in KDC medium containing ¹³C-labeled glucose and the L-glutamic acid accumulated in the medium was analyzed by LC-MS, all of the L-glutamic acid accumulated by NG13-pCCS contained ¹³C, whereas all of the L-glutamic acid accumulated by NG13-pKCT contained ¹²C. In other words, the L-glutamic acid produced by NG13-pKCT was converted from citric acid taken up from the KDC medium.

These results suggest that CS and citric acid:Na⁺ symporter independently contribute to production of L-glutamic acid. Then, NG13-pCCS-pKCT having both plasmid pCCS and pKCT was constructed,. As a control, NG13-pCCS-pCDF having both plasmid pCCS and pCDFDuet-1 (empty vector) was also constructed. Both strains were examined for L-glutamic acid productivity in the static culture for 5 days at 25°C in KDC medium. The results are shown in Table 10.

### [Table 10]

**Table 10**

| Strain | Cultu re me dium | Maximum nitroge n fixing ability (*µ*mol/4-h/tub e) | Final bacteri a count (10⁹ cells/tu be) | L-glutamic acid concentr ation in Day5 (mg/L) |
|---|---|---|---|---|
| NG 13-pCCS-p CDF | KDC | 0.26 | 0.69 | 120.9 |
| NG 13-pCCS-p KCT | KDC | 0.26 | 0.53 | 166.6 |

Compared to the maximum nitrogen fixing ability, final bacteria count and L-glutamic acid productivity when the NG13-pCCS shown in Table 8 was cultured in KDC medium, the maximum nitrogen fixing ability was reduced to about half and the final bacteria count was largely unchanged when NG13-pCCS-pCDF was cultured in KDC medium. L-glutamic acid productivity also showed no significant change. When NG13-pCCS-pKCT was cultured in KDC medium, the maximum nitrogen fixing ability and final bacteria count were almost same as those of NG13-pCCS-pCDF, however the L-glutamic acid productivity was greatly increased. Considering the values for L-glutamic acid productivity with citric acid:Na⁺ symporter shown in Table 9, the effect of CS and the effect of citric acid:Na⁺ symporter for L-glutamic acid productivity appear to function additively.

Media based on the composition of KDC medium with double the amount of glucose or sodium citrate (KDDC medium comprising 15 g/L glucose and 7.5 g/L sodium citrate, KDCC medium comprising 7.5 g/L glucose and 15 g/L sodium citrate, and KDDCC medium comprising 15 g/L glucose and 15 g/L sodium citrate) were prepared, and 25 mL of each was placed in a φ90 × 15 mm sterilized dish. NG13-pCCS-pKCT was inoculated into each dishat 10⁷ cells and statically cultured for 17 days at 25°C in an environment with humidity maintained not to evaporate the medium, and the L-glutamic acid productivity was examined. The results are shown in Table 11.

### [Table 11]

**Table 11**

| Strain | Culture medium | L-glutamic acid concentration in D ay17 (mg/L) |
|---|---|---|
| NG13-pCCS-pKCT | KD | 63 |
| NG13-pCCS-pKCT | KDC | 619 |
| NG13-pCCS-pKCT | KDDC | 1075 |
| NG13-pCCS-pKCT | KDCC | 557 |
| NG13-pCCS-pKCT | KDDCC | 690 |

The L-glutamic acid production compared with KD medium were approximately 9.8-fold for KDC medium, approximately 17-fold for KDDC medium, approximately 8.8-fold for KDCC medium and approximately 11-fold for KDDCC medium. Increasing the area of the culture contacting with air in a dish can drastically improve L-glutamic acid productivity.

This method of directly using nitrogen molecules in the atmosphere and citric acid in medium for fermentative production of L-glutamic acid, by increasing the production of citric acid:Na⁺ symporter in diazotrophically grown NG13, is an invention based on entirely new idea. Moreover, the method of fermentative production of L-glutamic acid augmented by an additive effect, adding the overproduction of citric acid:Na⁺ symporter to the known technology of producing L-glutamic acid by overproduction of CS, is likewise an invention based on entirely new idea. Since citric acid is an inexpensive organic acid, the present invention can provide an alternative method to current L-glutamic acid fermentative production which depends on the Haber-Bosch method.

### Example 7: Enhancement of nitrogen fixing ability in nitrogen-fixing bacteria other than NG13

Media were prepared by switching the 7.5 g/L glucose of KD and KDC medium to 3.75 g/L glucose and 3.75 g/L fructose, and used for culturing of various nitrogen-fixing bacteria of the phylum Proteobacteria other than NG13, as listed in Table 12. After static culture at 25°C for 24 hours, acetylene was introduced with sealed by butyl rubber and the nitrogen fixing ability was calculated from the amount of ethylene production at 168 hours after culturing. The results are shown in Table 12.

### [Table 12]

**Table 12**

| Strain | Enhancement rate for nitrogen fixing abili ty compared to citric acid-free culturing |
|---|---|
| Xanthobacter sp. HB61 | 3.5 |
| Azorhizobium caulinodans JCM20966 | 9.6 |
| Rahnella aquatilis JC1683 | 9.3 |
| Rahnella aquatilis JC1687 | 1.3 |
| Kosakonia sp. HB92 | 3.9 |

Enhanced nitrogen fixing ability was also observed by addition of citric acid to medium for *Rahnella aquatilis* JCM1683, *Raoultella terrigena* JCM1687, and *Kosakonia* sp. HB92 belonging to the same Gammaproteobacteria class as NG13, as well as *Xanthobacter* sp. HB61 and *Azorhizobium caulinodans* JCM 20966 of a different class from NG13. These results suggest that a method of enhancing nitrogen fixing ability by addition of citric acid can be applied not only to NG13 but also to a wide range of nitrogen-fixing bacteria in the phylum Proteobacteria.

### Example 8: Fermentative production of L-glutamic acid by Raoultella terrigena JCM1687 with cotransferred genes of the citrate synthase and citrate transporter

*Raoultella terrigena* JCM1687 (R. terrigena-pCCS) having a plasmid pCCS which expresses the CS gene of *Corynebacterium glutamicum* was constructed in the same manner as NG13. The modified strains were cultured by static culture for 6 days at 25°C in KD medium and KDC medium with addition of IPTG to a final concentration of 0.02 mM, and the L-glutamic acid productivity of each was examined. The results are shown in Table 13.

### [Table 13]

**Table 13**

| Strain | Culture medium | Maximum nit rogen fixing a bility (*µ*mol/4-h/ tube) | Final bacteri a count (OD 600 nm) | L-glutamic acid concentr ation in Day6 (mg/L) |
|---|---|---|---|---|
| R. terrigena | KD | 0.16 | 0.26 | N.D. |
| R. terrigena | KDC | 0.48 | 0.37 | N.D. |
| R. terrigena-pCCS | KD | 0.0060 | 0.10 | N.D. |
| R. terrigena-pCCS | KDC | 0.0062 | 0.09 | 21.8 |

Enhanced nitrogen fixing ability during culturing in KDC medium was observed with both strains R. terrigena and R. terrigena-pCCS. R. terrigena-pCCS also produced L-glutamic acid when cultured in KDC medium.

*Raoultella terrigena* JCM1687 (R. terrigena-pCCS-pKCT) having a plasmid pCCS which expresses the CS gene of *Corynebacterium glutamicum* and plasmid pKCT which expresses the citric acid:Na⁺ symporter gene of NG13, was constructed. The modified strains were cultured by static culture for 6 days at 25°C in KDD medium and KDC medium with addition of IPTG to a final concentration of 0.02 mM, and the L-glutamic acid productivity of each was examined. The results are shown in Table 14.

### [Table 14]

**Table 14**

| Strain | Culture medi um | L-glutamic acid concentration inDay 6 (mg/L) |
|---|---|---|
| R. terrigena | KDD | N.D. |
| R. terrigena | KDC | N.D. |
| R. terrigena-pCCS-pKCT | KDD | N.D. |
| R. terrigena-pCCS-pKCT | KDC | 93 |

Accumulation of L-glutamic acid was observed when R. terrigena-pCCS-pKCT, having cotransferred genes of citrate synthase and citrate transporter, was cultured in KDC medium.

KDC medium and KDDC medium were prepared, 25 mL of each was placed in a φ90 × 15 mm sterilized dish, and R. terrigena-pCCS-pKCT was inoculated into the dish. Static culture was carried out for 6 days at 25°C in an environment with humidity maintained not to evaporate the medium, and the L-glutamic acid productivity was examined. The results are shown in Table 15.

### [Table 15]

**Table 15**

| Strain | Culture medi um | L-glutamic acid concentration inDay 6 (mg/L) |
|---|---|---|
| R. terrigena-pCCS-pKCT | KDD | 163 |
| R. terrigena-pCCS-pKCT | KDDC | 168 |

L-glutamic acid was also produced and the amount was increased by dish culturing of R. terrigena-pCCS-pKCT. This result demonstrated that the method of the invention for producing L-glutamic acid is not limited to *Klebsiella* bacteria.

### Example 9: Fermentative production of L-glutamic acid by Klebsiella variicola JCM12419 with cotransferred genes of the citrate synthase and citrate transporter

*Klebsiella variicola* JCM12419, havinga plasmid pCCS which expresses the CS gene of *Corynebacterium glutamicum* (K. variicola-pCCS), *Klebsiella variicola* JCM12419, havinga plasmid pKCT which expresses the citric acid:Na⁺ symporter gene of NG13 (K. variicola-pKCT), and *Klebsiella variicola* JCM12419, having a plasmid pCCS and plasmid pKCT (K. variicola-pCCS-pKCT), were constructed in the same manner as NG13. The modified strains were statically cultured for 6 days at 25°C in KDD medium and KDC medium with addition of IPTG to a final concentration of 0.02 mM, and the L-glutamic acid productivity of each was examined. The results are shown in Table 16.

### [Table 16]

**Table 16**

| Strain | Culture mediu m | L-glutamic acid concentration i n Day6 (mg/L) |
|---|---|---|
| K. variicola | KDD | N.D. |
| K. variicola | KDC | N.D. |
| K. variicola-pCCS | KDD | N.D. |
| K. variicola-pCCS | KDC | 81 |
| K. variicola-pKCT | KDD | N.D. |
| K. variicola-pKCT | KDC | 68 |
| K. variicola-pCCS-pKCT | KDD | 15 |
| K. variicola-pCCS-pKCT | KDC | 110 |

Production of L-glutamic acid was observed when K. variicola-pCCS, having a transferred gene of citrate synthase, K. variicola-pKCT, having a transferred gene of citrate transporter, and K. variicola-pCCS-pKCT, having cotransferred genes of both citrate synthase and citrate transporter, were cultured in KDC medium.

To confirm that the nitrogen atom taken up into the produced L-glutamic acid by K. variicola-pCCS was from nitrogen molecules in the atmosphere, K. variicola-pCCS was cultured for 6 days in the KDC medium with the top of the test tube sealed with a butyl rubber and the air layer was replaced with mixed gas of ¹⁵N₂ and O₂ (¹⁵N₂:O₂ = 82:18). L-glutamic acid accumulated at 92 mg/L in the medium in 6 days of culturing was analyzed by LC-MS. LC-MS analysis showed that the majority consisted of L-glutamic acid cation ([C₅H₁₀¹⁵NO₄]⁺) with a molecular weight of 149, containing ¹⁵N. This showed that the nitrogen atom of L-glutamic acid produced by K. variicola-pCCS in the KDC medium was mostly derived from nitrogen molecules in the atmosphere by nitrogen fixation, similar to NG13.

KDC medium and KDDC medium were prepared, 20 mL of each was placed in a φ90 × 15 mm sterilized dish, and K. variicola-pCCS-pKCT was inoculated into the dish. Static culture was carried out for 14 days at 25°C in an environment with humidity maintained not to evaporate the medium, and the L-glutamic acid productivity was examined. The results are shown in Table 17.

### [Table 17]

**Table 17**

| Strain | Culture mediu m | L-glutamic acid concentration i n Day14 (mg/L) |
|---|---|---|
| K. variicola-pCCS-pKCT | KDC | 506 |
| K. variicola-pCCS-pKCT | KDDC | 1095 |

With K. variicola-pCCS-pKCT as well, productivity of L-glutamic acid was vastly improved by dish culturing.

### Example 10: Fermentative production of L-glutamic acid by Klebsiella sp.NBRC100048 and Klebsiella sp.NBRC109911 having cotransferred genes of the citrate synthase and citrate transporter

*Klebsiella* sp.NBRC100048 (Klebsiella sp. NBRC100048-pCCS-pKCT) and *Klebsiella* sp.NBRC109911 (Klebsiella sp. NBRC109911-pCCS-pKCT), having a plasmid pCCS which expresses the CS gene of *Corynebacterium glutamicum* and a plasmid pKCT which expresses the citric acid:Na⁺ symporter gene of NG13, were constructed. The modified strains were statically cultured for 6 days at 25°C in KDD medium and KDC medium with addition of IPTG to a final concentration of 0.02 mM, and the L-glutamic acid productivity of each was examined. The results are shown in Table 18.

### [Table 18]

### Table 18

**Table 18**

| Strain | Culture mediu m | L-glutamic acid concentration i n Day6 (mg/L) |
|---|---|---|
| Klebsiella sp. NBRC100048 | KDD | N.D. |
| Klebsiella sp. NBRC100048 | KDC | N.D. |
| Klebsiella sp. NBRC100048-pCCS-pKCT | KDD | N.D. |
| Klebsiella sp. NBRC100048-pCCS-pKCT | KDC | 166 |
| Klebsiella sp. NBRC109911 | KDD | N.D. |
| Klebsiella sp. NBRC109911 | KDC | N.D. |
| Klebsiella sp. NBRC109911-pCCS-pKCT | KDD | N.D. |
| Klebsiella sp. NBRC109911-pCCS-pKCT | KDC | 170 |

A large amount of L-glutamic acid was accumulated in KDC medium with both of these *Klebsiella* strains as well when pCCS and pKCT were transferred to both strains, similar to *Klebsiella oxytoca* and *Klebsiella variicola* described above.

### Example 11: Fermentative production of L-glutamic acid by Rahnella aquatilis JCM1683 having cotransferred genes of the citrate synthase and citrate transporter

*Rahnella aquatilis* JCM1683 (R. aquatilis-pCCS-pKCT) having a plasmid pCCS which expresses the CS gene of *Corynebacterium glutamicum* and a plasmid pKCT which expresses the citric acid:Na⁺ symporter gene of NG13, was constructed. The modified strains were statically cultured for 6 days at 25°C in KDC medium with addition of IPTG to a final concentration of 0.02 mM, and the L-glutamic acid productivity was examined. The results are shown in Table 19.

### [Table 19]

**Table 19**

| Strain | Cultu re me dium | L-glutamic acid concentration in Day6 (mg/L) |
|---|---|---|
| R. aquatilis | KDC | N.D. |
| R. aquatilis -pC CS-pKCT | KDC | 53.5 |

Production of L-glutamic acid was observed when R. aquatilis-pCCS-pKCT, having cotransferred genes of citrate synthase and citrate transporter, was cultured in KDC medium.

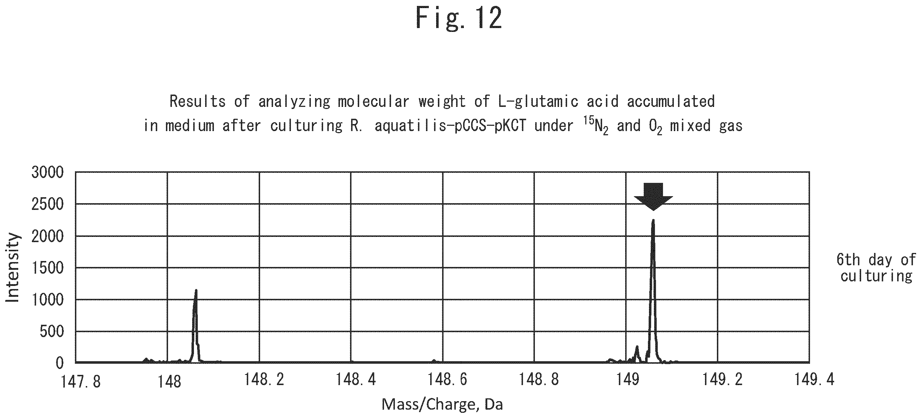

To confirm that the nitrogen atom taken up into the produced L-glutamic acid by R. aquatilis-pCCS-pKCT was from nitrogen molecules in the atmosphere, R. aquatilis-pCCS-pKCT was cultured for 6 days in the KDC medium with the top of the test tube sealed with a butyl rubber and the air layer was replaced with mixed gas of ¹⁵N₂ and O₂ (¹⁵N₂:O₂ = 82:18). L-glutamic acid accumulated at 41 mg/L in the medium in 6 days of culturing was analyzed by LC-MS. LC-MS analysis showed that the majority consisted of L-glutamic acid cation ([C₅H₁₀¹⁵NO₄]⁺) with a molecular weight of 149, containing ¹⁵N (Fig. 12). This showed that the nitrogen atom of L-glutamic acid produced by R. aquatilis-pCCS-pKCT in the KDC medium was mostly derived from nitrogen molecules in the atmosphere by nitrogen fixation, similar to NG13.

These results demonstrate that the method for fermentative producing L-glutamic acid using nitrogen molecules in the atmosphere according to the invention can be carried out using nitrogen-fixing bacteria belonging to Enterobacteriaceae bacteria other than NG13.

### Example 12: Fermentative production of L-alanine and L-valine by NG13 having cotransferred genes of the citrate synthase gene, glutamate-pyruvate aminotransferase gene and L-alanine transporter gene

Glutamate-pyruvate aminotransferase is an enzyme that catalyzes a reaction producing L-alanine and 2-oxoglutaric acid from L-glutamic acid and pyruvic acid, and thus contributes to biosynthesis of L-alanine. It is thought that transferring the glutamate-pyruvate aminotransferase gene into NG13-pCCS leads to transfer of the amino residue of L-glutamic acid produced using nitrogen molecules in the atmosphere to pyruvic acid, thereby producing L-alanine or its salt. It is further thought that transferring the L-alanine transporter gene, which has the function of transporting L-alanine out of cells, enhances extracellular transport of produced L-alanine and thus improves productivity of L-alanine or its salt. A strain having both genes cotransferred was therefore constructed, and the L-amino acid present in the culture of the strain was examined.

### (1) Construction of plasmid pKACE

The glutamate-pyruvate aminotransferase gene and the L-alanine transporter gene of NG13 were cloned based on genomic information of *Klebsiella oxytoca* M5a1, and their sequence information was acquired (SEQ ID NO: 33 and SEQ ID NO: 34). The procedure for constructing plasmid pKACE expressing both the glutamate-pyruvate aminotransferase gene and L-alanine transporter gene was as follows. The primers shown in Table 4 were used for PCR in combination with the templates and primers shown in Table 5, and both gene fragments were amplified. The primers were designed so that an approximately 20 bp sequence homologous to the vector was added to both ends, and so that the introduced gene was expressed using the expression promoter and ribosome binding site (RBS) in the vector (Table 4). The fragment of the vector was also prepared by PCR. The target gene fragments and vector fragments were ligated by the Gibson Assembly. A 10 µL portion of reaction mixture containing both fragments was used to transform *E. coli* DH5α, and a drug selection marker in the plasmids was used to screen the colonies. The plasmids were extracted from the appearing colonies and subjected to sequence analysis to confirm that the plasmids were correctly designed (Table 5).

### (2) Introduction of plasmid pKACE into NG13-pCCS

NG13-pCCS was transformed by plasmid pKACE using electroporation, and the transformants were separated by chloramphenicol resistance. Plasmids were prepared from the strain, and restriction enzyme treatment and subsequent agarose gel electrophoresis were carried out to confirm that the target plasmid was retained.

### (3) Culturing of NG13-pCCS-pKACE

NG13-pCCS, having a plasmid pCCS which expresses the citrate synthase gene of *Corynebacterium glutamicum,* and NG13-pCCS-pKACE, having a plasmid pKACE which co-expresses the glutamate-pyruvate aminotransferase gene and L-alanine transporter gene of NG13, were statically cultured for 7 days at 25°C in KDC medium with addition of IPTG to a final concentration of 0.1 mM, and the L-amino acid productivity was examined. The results are shown in Table 20.

### [Table 20]

**Table 20**

| Strain | Cultu re me dium | L-glutamic acid con centration in Day7 (mg/L) | L-alanine concent ration in Day7 (mg /L) | L-valine concentra tion in Day7 (mg/ L) |
|---|---|---|---|---|
| NG13-pCCS | KDC | 62 | N.D. | N.D. |
| NG 13-pCCS-p KACE | KDC | 9.2 | 27.9 | 15.1 |

Upon analyzing L-amino acid accumulated in the culture of NG13-pCCS-pKACE, having a plasmid pCCS with a plasmid pKACE, the target substance L-alanine was detected in addition to L-glutamic acid. L-valine was detected as well. *Klebsiella* bacteria produce L-valine-pyruvate aminotransferase which catalyzes the following reaction: 3-methyl-2-oxobutanoate + L-alanine = L-valine + pyruvate. When *Klebsiella* bacteria are grown diazotrophically, normally L-alanine is produced by glutamate-pyruvate aminotransferase from L-glutamic acid derived from nitrogen in the air, and L-valine is produced from the L-alanine by L-valine-pyruvate aminotransferase. In NG13-pCCS, therefore, L-alanine production by glutamate-pyruvate aminotransferase from L-glutamic acid whose production certainly increases, and L-valine production by L-valine-pyruvate aminotransferase should be increased some extent, but presumably do not reach a level such as to be produced into the medium. In contrast, overproduction of glutamate-pyruvate aminotransferase in NG13-pCCS results in extremely enhanced production of L-alanine, whereby production of L-valine by L-valine-pyruvate aminotransferase is concomitantly enhanced, and presumably as a result, both are produced into the medium. It was thus verified that using NG13-pCCS-pKACE achieved fermentative production of L-alanine with L-valine, whose nitrogen atoms were derived from nitrogen in the air.

These results demonstrate that the method for fermentative production of L-glutamic acid using nitrogen molecules in the atmosphere according to the invention can also be used for fermentative production of amino acids other than L-glutamic acid.

### INDUSTRIAL APPLICABILITY

The microorganisms of the invention are to be used in the field of fermentative production of L-glutamic acid or its salt. Using nitrogen in the atmosphere makes it possible to be independent of the use of nitrogen compounds produced by the conventional Haber-Bosch method, which is consuming large amounts of energy.

### [Accession Nos.]

Accession No.: NITE BP-03721 (deposit number: NITE ABP-03721, date of receipt: July 21, 2023, depositary institution name and address: NITE Patent Microorganisms Depositary (NPMD) of the independent administrative National Institute of Technology and Evaluation, 2-5-8 Kazusa Kamatari, Room 122, Kisarazu City, Chiba Prefecture, Japan 292-0818.

[Sequence Listing]

## Claims

1. A method for fermentatively producing L-glutamic acid or its salt from nitrogen molecules in the atmosphere, comprising culturing nitrogen-fixing bacteria to which at least one gene selected from the group consisting of citrate transporter, citrate synthase and 2-methylcitrate synthase has been introduced, in nitrogen limited medium containing an organic acid or its salt forming the TCA cycle.

2. The method according to claim 1, wherein the nitrogen limited medium contains a nitrogen source at 3 mg/L to 1500 mg/L of yeast extract equivalent and/or 0.02 mM to 15 mM of ammonium ion equivalent.

3. The method according to claim 1, wherein the organic acid or its salt is at a concentration of 0.5 g/L to 100 g/L.

4. The method according to claim 3, wherein the organic acid is citric acid, succinic acid, malic acid, fumaric acid or 2-oxoglutaric acid.

5. The method according to any one of claims 1 to 4, wherein the nitrogen-fixing bacteria are microorganisms of Enterobacteriaceae.

6. The method according to claim 5, wherein the nitrogen-fixing bacteria are microorganisms selected from the group consisting of *Klebsiella, Rahnella, Raoultella* and *Kosakonia* bacteria.

7. Nitrogen-fixing bacteria selected from the group consisting of *Klebsiella, Rahnella, Kosakonia* and *Raoultella,* wherein the citrate transporter gene have been introduced thereto.

8. Nitrogen-fixing bacteria according to claim 7, wherein the nitrogen-fixing bacteria of *Klebsiella* are selected from the group consisting of *Klebsiella oxytoca, Klebsiella michiganensis, Klebsiella grimontii, Klebsiella pasteurii, Klebsiella pneumoniae, Klebsiella variicola and Klebsiella indica,* the nitrogen-fixing bacteria of *Rahnella* is *Rahnella aquatilis,* or the nitrogen-fixing bacteria of *Raoultella* are selected from the group consisting of *Raoultella terrigena, Raoultella ornithinolytica* and *Raoultella planticola.*

9. Nitrogen-fixing bacteria according to claim 7, wherein the nitrogen-fixing bacteria are *Klebsiella oxytoca* NG13 (Accession No.: NITE BP-03721), *Klebsiella indica* (JCM33718), *Klebsiella variicola* (JCM12419), Klebsiella sp. (NBRC100048, NBRC100441, NBRC109911), *Raoultella terrigena* (JCM1687 = ATCC33257), *Rahnella aquatilis* (JCM1683 = ATCC33071) and *Klebsiella planticola* (JCM20069 = ATCC8329).

10. Nitrogen-fixing bacteria according to any one of claims 7 to 9, wherein the nitrogen-fixing bacteria fermentatively produces L-glutamic acid or its salt from nitrogen molecules under nitrogen limitation in the atmosphere.
